# EUROPEAN PATENT SPECIFICATION

(11) **EP 4 034 193 B1**
(45) Date of publication and mention of the grant of the patent: **05.11.2025**
(21) Application number: 20786340.8
(22) Date of filing: 24.09.2020
(51) Int. Cl.: A61M 5/142, A61M 5/20, A61M 15/00, A61M 5/315, A61M 11/00, A61M 15/08, G16H 40/67, G16H 20/17

(54) **DRUG ADMINISTRATION SYSTEM CONFIGURED TO DETERMINE A DRUG DOSING SCHEME**
MEDIKAMENTENVERABREICHUNGSSYSTEM, KONFIGURIERT IST ZUM BESTIMMEN EINES MEDIKAMENTENDOSIERUNGSSCHEMAS
SYSTÈME D'ADMINISTRATION DES MÉDICAMENTS CONFIGURÉ POUR DÉTERMINER UN SCHÉMA DE DOSAGE DES MÉDICAMENTS

(30) Priority: 25.09.2019 US 201962905463 P; 06.05.2020 US 202063020947 P
(43) Date of publication of application: 03.08.2022
(73) Proprietor: Janssen Pharmaceuticals, Inc., Titusville, NJ 08560 (US)
(72) Inventor: ALBERTINI, Francesco N., Milpitas, California 95035 (US); BAKOS, Gregory J., Cincinnati, Ohio 45242 (US); DIUBALDI, Anthony R., Spring House, Pennsylvania 19477 (US); HARRIS, Jason L., Cincinnati, Ohio 45242 (US); HUTCHINSON, Michael, Malvern, Pennsylvania 19355 (US); LEQUIEU, Wouter Jacques Noel, 2340 Beerse (BE); SHELTON IV, Frederick E., Cincinnati, Ohio 45242 (US); SZABO, George, Spring House, Pennsylvania 19477 (US); VESOLE, Steven M., Milpitas, California 95035 (US)
(74) Representative: Carpmaels & Ransford LLP
(86) International application number: PCT/IB2020/058970
(87) International publication number: WO 2021/059214

(56) References cited:
- EP-A1- 2 572 740
- WO-A1-2009/053914
- WO-A1-2009/081262
- WO-A1-2019/099568
- WO-A1-2019/156639
- JP-A- 2020 062 420
- US-A1- 2010 100 038
- US-A1- 2011 275 986
- US-A1- 2011 319 322
- US-A1- 2019 164 641
- US-A1- 2019 180 856
- US-B2- 8 600 682
- US-B2- 9 220 456

## Description

### FIELD

The embodiments described herein relate to a device for administering and/or provision of a drug. The present disclosure further relates to a system in which the device can be used, and a method of administration, and a further method associated with the system.

### BACKGROUND

Pharmaceutical products (including large and small molecule pharmaceuticals, hereinafter "drugs") are administered to patients in a variety of different ways for the treatment of specific medical indications. Regardless of the manner of the administration, care must be taken when administering drugs to avoid adverse effects on the patient. For example, care must be taken not to administer more than a safe amount of the drug to the patient. This requires consideration of the amount of dose given and the time frame over which the dose is delivered, sometimes in relation to previous doses, or doses of other drugs. Moreover, care must be taken not to inadvertently administer an incorrect drug to the patient, or drugs that have degraded due to their age or storage conditions. All of these considerations can be conveyed in guidance associated with the specific drugs or drug combinations. However, this guidance is not always followed correctly, for example due to mistakes, such as human error. This can lead to adverse effects on the patient or result in inappropriate drug administration, for example insufficient or excessive volume of drug being administered for the specific medical indication.

Drugs are typically administered to patients according to a drug dosing scheme. Drug dosing schemes can specify a volume of drug to be administered, the frequency of drug administration, the time of drug administration, etc. Typically, a doctor or other healthcare professional will establish a starting drug dosing scheme for a patient based on dosing data such as patient body weight, height and age. However, this starting drug dosing scheme may not be optimal and, further, will not take account of factors which affect the patient's response to the drug dosing scheme but which also change over time. Therefore, the patient may have to monitor both their response to the drug dosing scheme and factors which may affect their response in order to work extensively with doctors to optimize the drug dosing scheme and so ensure it is effective.

Optimization of drug dosing schemes relies heavily on patient compliance with the starting drug dosing scheme and accurate, regular monitoring of patient symptoms. Furthermore, in some cases, the patient may be required to monitor a large number of factors and take all of these factors into account to calculate dosing scheme adjustments. If the patient fails to take proper account of all the necessary factors or does not monitor their symptoms properly, a drug dosing scheme may not be adjusted accordingly and so may not be effective.

In relation to how a drug is administered to the patient, there are various dosage forms that can be used. For example, these dosage forms may include parenteral, inhalational, oral, ophthalmic, nasal topical, and suppository forms of one or more drugs.

The dosage forms can be administered directly to the patient via a drug administration device. There are a number of different types of drug administration devices commonly available for delivery of the various dosage forms including: syringes, injection devices (e.g., autoinjectors, jet injectors, and infusion pumps), nasal spray devices, and inhalers. Pertinent devices are disclosed in US2019/180856 A1 and EP 2572740 A1.

### SUMMARY

The invention is defined by the subject-matter of independent claims 1 and 2. Further embodiments are defined in the dependent claims. **In** one aspect, a drug administration system is provided that in one embodiment includes a drug administration device that includes a communications interface configured to communicate with a communications interface of a computer system. The drug administration device also includes at least one sensor configured to obtain sensor data and communicate the sensor data to the drug administration device. The drug administration device is configured to utilize a first drug dosing scheme when a drug is delivered from the drug administration device to a patient, and determine a second drug dosing scheme for delivering the drug from the drug administration device to the patient. The second drug dosing scheme is dependent on dosing data from the computer system, and the sensor data from the at least one sensor. The sensor data is obtained by the at least one sensor during and/or after the delivery of the drug according to the first drug dosing scheme. The drug administration device is also configured to utilize the second drug dosing scheme when the drug is delivered from the drug administration device to the patient.

The drug administration device can vary in any number of ways. For example, the drug administration device can be configured to determine the first drug dosing scheme for delivering the drug to the patient before utilizing the first drug dosing scheme when the drug is delivered from the drug administration device to the patient. For yet another example, each of the first and second drug dosing schemes can each specify one or more of the following drug dosing parameters: drug delivery rate, drug delivery time and/or date, drug delivery volume, and drug delivery duration. For another example, the at least one sensor can include one or more of a patient monitoring sensor configured to obtain sensor data relating to the condition of the patient, a device sensor configured to obtain sensor data relating to the drug administration device, an environment sensor configured to obtain sensor data relating to an environment in which the drug administration device is present, and a location sensor configured to obtain sensor data relating to geographical location of the drug administration device. For yet another example, the drug administration system can also include at least one external sensor configured to obtain external sensor data and communicate the external sensor data to the computer system, and the second drug dosing scheme can be further dependent on external sensor data from the computer system. For still another example, the drug administration system can also include a second drug administration device including a communications interface configured to communicate with the communications interface of the computer system, the drug administration can also include at least one sensor configured to obtain supplementary sensor data and communicate the supplementary sensor data to the second drug administration device, and the second drug dosing scheme can be further determined dependent on the supplementary sensor data from the computer system.

For another example, the drug administration system can also include a second drug administration device including a communications interface configured to communicate with the communications interface of the computer system, the drug administration can also include at least one user interface configured to obtain supplementary user input data and communicate the supplementary user input data to the second drug administration device, and the second drug dosing scheme can be further determined dependent on the supplementary user input data from the computer system. In at least some embodiments, the system can also include at least one sensor configured to obtain supplementary sensor data and communicate the supplementary sensor data to the second drug administration device, and the second drug dosing scheme can be further determined dependent on the supplementary sensor data from the computer system. The at least one sensor can be configured to obtain supplementary sensor data that includes one or more of: a patient monitoring sensor configured to obtain supplementary sensor data relating to a condition of a patient, a device sensor configured to obtain supplementary sensor data relating to the second drug administration device, an environment sensor configured to obtain supplementary sensor data relating to an environment in which the second drug administration device is present, and a location sensor configured to obtain supplementary sensor data relating to geographical location of the second drug administration device.

For another example, the drug administration device can be an autoinjector, and the first and second drug dosing schemes can each specify one or more of the following dosing parameters: a discharge nozzle advance depth of a discharge nozzle during delivery of the drug to the patient, a discharge nozzle velocity of the discharge nozzle during delivery of the drug to the patient, and a discharge nozzle acceleration of the discharge nozzle during delivery of the drug to the patient. For yet another example, the drug administration device can be an inhaler. For still another example, the drug administration device can be an infusion pump.

For another example, the first drug dosing scheme can be determined dependent on dosing data communicated from the computer system. For yet another example, the first and second drug dosing schemes can each specify one or more of drug delivery rate, drug delivery volume, and drug delivery duration, and a value of one or more of the parameters in the first drug dosing scheme is different than a value of the corresponding parameter in the second drug dosing scheme.

For another example, the drug can include at least one of infliximab, golimumab, ustekinumab, daratumumab, guselkumab, epoetin alfa, risperidone, esketamine, ketamine, and paliperidone palmitate.

In another embodiment, a drug administration system includes a drug administration device that includes a communications interface configured to communicate with a communications interface of a computer system. The system also includes at least one user interface configured to obtain user input data and communicate the user input data to the drug administration device The drug administration device is configured to utilize a first drug dosing scheme when a drug is delivered from the drug administration device to a patient, and determine a second drug dosing scheme for delivering the drug to the patient. The second drug dosing scheme is dependent on dosing data from the computer system, and the user input data from the at least one user interface. The user input data is obtained by the user interface during and/or after the delivery of the drug from the drug administration device according to the first drug dosing scheme. The drug administration device is also configured to utilize the second drug dosing scheme when the drug is delivered from the drug administration device to the patient.

The drug administration system can have any number of variations. For example, the drug administration device can be configured to determine the first drug dosing scheme for delivering the drug from the drug administration device to the patient before utilizing the first drug dosing scheme when the drug is delivered from the drug administration device to the patient. For another example, the system can include at least one sensor configured to obtain sensor data and communicate the sensor data to the drug administration device, the drug administration device can be configured to determine the second drug dosing scheme further dependent on the sensor data from the at least one sensor, and the sensor data can be obtained by the at least one sensor during and/or after the delivery of the drug from the drug administration device according to the first drug dosing scheme. For yet another example, each of the first and second drug dosing schemes can each specify one or more of the following drug dosing parameters: drug delivery rate, drug delivery time and/or date, drug delivery volume, and drug delivery duration.

For another example, the at least one sensor can include one or more of a patient monitoring sensor configured to obtain sensor data relating to the condition of the patient, a device sensor configured to obtain sensor data relating to the drug administration device, an environment sensor configured to obtain sensor data relating to an environment in which the drug administration device is present, and a location sensor configured to obtain sensor data relating to geographical location of the drug administration device.

For another example, the drug administration system can also include at least one external sensor configured to obtain external sensor data and communicate the external sensor data to the computer system, and the second drug dosing scheme can be further dependent on external sensor data from the computer system.

For still another example, the drug administration system can also include a second drug administration device including a communications interface configured to communicate with the communications interface of the computer system, the drug administration can also include at least one sensor configured to obtain supplementary sensor data and communicate the supplementary sensor data to the second drug administration device, and the second drug dosing scheme can be further determined dependent on the supplementary sensor data from the computer system.

For another example, the drug administration system can also include a second drug administration device including a communications interface configured to communicate with the communications interface of the computer system, the drug administration can also include at least one user interface configured to obtain supplementary user input data and communicate the supplementary user input data to the second drug administration device, and the second drug dosing scheme can be further determined dependent on the supplementary user input data from the computer system. In at least some embodiments, the system can also include at least one sensor configured to obtain supplementary sensor data and communicate the supplementary sensor data to the second drug administration device, and the second drug dosing scheme can be further determined dependent on the supplementary sensor data from the computer system. The at least one sensor can be configured to obtain supplementary sensor data that includes one or more of: a patient monitoring sensor configured to obtain supplementary sensor data relating to a condition of a patient, a device sensor configured to obtain supplementary sensor data relating to the second drug administration device, an environment sensor configured to obtain supplementary sensor data relating to an environment in which the second drug administration device is present, and a location sensor configured to obtain supplementary sensor data relating to geographical location of the second drug administration device.

For another example, the drug administration device can be an autoinjector, and the first and second drug dosing schemes can each specify one or more of the following dosing parameters: a discharge nozzle advance depth of a discharge nozzle during delivery of the drug to the patient, a discharge nozzle velocity of the discharge nozzle during delivery of the drug to the patient, and a discharge nozzle acceleration of the discharge nozzle during delivery of the drug to the patient. For yet another example, the drug administration device can be an inhaler. For still another example, the drug administration device can be an infusion pump.

For another example, the first drug dosing scheme can be determined dependent on dosing data communicated from the computer system. For yet another example, the first and second drug dosing schemes can each specify one or more of drug delivery rate, drug delivery volume, and drug delivery duration, and a value of one or more of the parameters in the first drug dosing scheme is different than a value of the corresponding parameter in the second drug dosing scheme.

For another example, the drug can include at least one of infliximab, golimumab, ustekinumab, daratumumab, guselkumab, epoetin alfa, risperidone, esketamine, ketamine, and paliperidone palmitate.

In another aspect, a method of determining a drug dosing scheme for delivering a drug to a patient is provided and in one embodiment includes delivering a drug to a patient according to a first drug dosing scheme using the drug administration device; obtaining sensor data using a sensor; determining, on the drug administration device, a second drug dosing scheme for delivering the drug from the drug administration device to the patient dependent on the sensor data and dosing data from a computer system; and delivering the drug to the patient according to the second drug dosing scheme using the drug administration device.

The method can have any number of variations. For example, the method can also include determining, on the drug administration device, a first drug dosing scheme for delivering the drug to the patient before delivering the drug to the patient according to the first drug dosing scheme.

For yet another example, each of the first and second drug dosing schemes can specify at least one of drug delivery rate, drug delivery volume, and drug delivery duration. For still another example, obtaining sensor data using the sensor comprises at least one of obtaining sensor data relating to a condition of the patient using a patient monitoring sensor, obtaining sensor data relating to the drug administration device using a device sensor, obtaining sensor data relating to an environment in which the drug administration device is present using an environment sensor, and obtaining sensor data relating to geographical location of the drug administration device using a location sensor. For another example, the method can include obtaining external sensor data using an external sensor, and determining the second drug dosing scheme for delivering the drug to the patient can include determining the second drug dosing scheme further dependent on the external sensor data. For yet another example, the method can include obtaining supplementary sensor data using a supplementary sensor, the method can include communicating the supplementary sensor data to a second drug administration device, and determining the second drug dosing scheme for delivering the drug to the patient can include determining the second drug dosing scheme for delivering the drug to the patient further dependent on the supplementary sensor data. Obtaining supplementary sensor data using the supplementary sensor can include at least one of obtaining supplementary sensor data relating to a condition of the patient using a patient monitoring sensor, obtaining supplementary sensor data relating to the drug administration device using a device sensor, obtaining supplementary sensor data relating to an environment in which the drug administration device is present using an environment sensor, and obtaining supplementary sensor data relating to geographical location of the drug administration device using a location sensor.

For another example, the method can include obtaining supplementary user input data using a user interface, the method can include communicating the supplementary user input a to the second drug administration device, and determining the second drug dosing scheme for delivering the drug to the patient can include determining the second drug dosing scheme for delivering the drug to the patient further dependent on the supplementary user input data. In at least some embodiments, the method can include obtaining supplementary sensor data using a supplementary sensor, the method can include communicating the supplementary sensor data to the second drug administration device, and determining the second drug dosing scheme for delivering the drug to the patient can include determining the second drug dosing scheme for delivering the drug to the patient further dependent on the supplementary sensor data. Obtaining supplementary sensor data using the supplementary sensor can include at least one of obtaining supplementary sensor data relating to a condition of the patient using a patient monitoring sensor, obtaining supplementary sensor data relating to the drug administration device using a device sensor, obtaining supplementary sensor data relating to an environment in which the drug administration device is present using an environment sensor, and obtaining supplementary sensor data relating to geographical location of the drug administration device using a location sensor.

For still another example, the drug administration device can be an autoinjector, and determining the first and second drug dosing schemes comprises specifying one or more of a discharge nozzle advance depth of a discharge nozzle for delivery of the drug to the patient, a discharge nozzle velocity of the discharge nozzle for delivery of the drug to the patient, and a discharge nozzle acceleration of the discharge nozzle for delivery of the drug to the patient. For yet another example, the drug delivery device can be an inhaler. For another example, the drug delivery device can be an infusion pump.

For another example, the method can include communicating dosing data from a computer system to the drug administration device, and determining, on the drug administration device, a first drug dosing scheme for delivering the drug to the patient dependent on the dosing data. For yet another example, the first and second drug dosing schemes can specify one or more of the following drug dosing parameters: drug delivery rate, drug delivery volume, and drug delivery duration, and a value of one or more of the parameters in the first drug dosing scheme is different than a value of the corresponding parameter in the second drug dosing scheme.

For another example, the drug can include at least one of infliximab, golimumab, ustekinumab, daratumumab, guselkumab, epoetin alfa, risperidone, esketamine, ketamine, and paliperidone palmitate.

In another embodiment, a method of determining a drug dosing scheme for delivering a drug to a patient includes delivering a drug to a patient according to the first drug dosing scheme using a drug administration device; obtaining user input data using a user interface; determining, on the drug administration device, a second drug dosing scheme for delivering a drug from the drug administration device to a patient dependent on the user input data and dosing data from a computer system; and delivering the drug to the patient according to the second drug dosing scheme using the drug administration device.

The method can vary in any number of ways. For example, the method can include determining, on the drug administration device, a first drug dosing scheme for delivering the drug to the patient before delivering the drug to the patient according to the first drug dosing scheme. For another example, the method can include obtaining sensor data using a sensor, and determining the second drug dosing scheme for delivering the drug to the patient further dependent on the sensor data.

For yet another example, each of the first and second drug dosing schemes can specify at least one of drug delivery rate, drug delivery volume, and drug delivery duration. For still another example, obtaining sensor data using the sensor comprises at least one of obtaining sensor data relating to a condition of the patient using a patient monitoring sensor, obtaining sensor data relating to the drug administration device using a device sensor, obtaining sensor data relating to an environment in which the drug administration device is present using an environment sensor, and obtaining sensor data relating to geographical location of the drug administration device using a location sensor. For another example, the method can include obtaining external sensor data using an external sensor, and determining the second drug dosing scheme for delivering the drug to the patient can include determining the second drug dosing scheme further dependent on the external sensor data. For yet another example, the method can include obtaining supplementary sensor data using a supplementary sensor, the method can include communicating the supplementary sensor data to a second drug administration device, and determining the second drug dosing scheme for delivering the drug to the patient can include determining the second drug dosing scheme for delivering the drug to the patient further dependent on the supplementary sensor data. Obtaining supplementary sensor data using the supplementary sensor can include at least one of obtaining supplementary sensor data relating to a condition of the patient using a patient monitoring sensor, obtaining supplementary sensor data relating to the drug administration device using a device sensor, obtaining supplementary sensor data relating to an environment in which the drug administration device is present using an environment sensor, and obtaining supplementary sensor data relating to geographical location of the drug administration device using a location sensor.

For another example, the method can include obtaining supplementary user input data using a user interface, the method can include communicating the supplementary user input a to the second drug administration device, and determining the second drug dosing scheme for delivering the drug to the patient can include determining the second drug dosing scheme for delivering the drug to the patient further dependent on the supplementary user input data. In at least some embodiments, the method can include obtaining supplementary sensor data using a supplementary sensor, the method can include communicating the supplementary sensor data to the second drug administration device, and determining the second drug dosing scheme for delivering the drug to the patient can include determining the second drug dosing scheme for delivering the drug to the patient further dependent on the supplementary sensor data. Obtaining supplementary sensor data using the supplementary sensor can include at least one of obtaining supplementary sensor data relating to a condition of the patient using a patient monitoring sensor, obtaining supplementary sensor data relating to the drug administration device using a device sensor, obtaining supplementary sensor data relating to an environment in which the drug administration device is present using an environment sensor, and obtaining supplementary sensor data relating to geographical location of the drug administration device using a location sensor.

For still another example, the drug administration device can be an autoinjector, and determining the first and second drug dosing schemes comprises specifying one or more of a discharge nozzle advance depth of a discharge nozzle for delivery of the drug to the patient, a discharge nozzle velocity of the discharge nozzle for delivery of the drug to the patient, and a discharge nozzle acceleration of the discharge nozzle for delivery of the drug to the patient. For yet another example, the drug delivery device can be an inhaler. For another example, the drug delivery device can be an infusion pump.

For another example, the method can include communicating dosing data from a computer system to the drug administration device, and determining, on the drug administration device, a first drug dosing scheme for delivering the drug to the patient dependent on the dosing data. For yet another example, the first and second drug dosing schemes can specify one or more of the following drug dosing parameters: drug delivery rate, drug delivery volume, and drug delivery duration, and a value of one or more of the parameters in the first drug dosing scheme is different than a value of the corresponding parameter in the second drug dosing scheme.

For another example, the drug can include at least one of infliximab, golimumab, ustekinumab, daratumumab, guselkumab, epoetin alfa, risperidone, esketamine, ketamine, and paliperidone palmitate.

### BRIEF DESCRIPTION OF DRAWINGS

The present invention is described by way of reference to the accompanying figures which are as follows:
Fig. 1 is a schematic view of a first type of drug administration device, namely an autoinjector;
Fig. 2 is a schematic view of a second type of drug administration device, namely an infusion pump;
Fig. 3 is a schematic view of a third type of drug administration device, namely an inhaler;
Fig. 4 is a schematic view of a fourth type of drug administration device, namely a nasal spray device;
Fig. 5A is a schematic view of a general drug administration device;
Fig. 5B is a schematic view of a universal drug administration device;
Fig. 6 is a schematic view of a housing for a dosage form;
Fig. 7 is a schematic view of one embodiment of a communication network system with which the drug administration devices and housing can operate;
Fig. 8 is a schematic view of one embodiment of a computer system with which the drug administration devices and housing can operate;
Fig. 9 is a schematic view of one embodiment of a drug administration system;
Fig. 10A is a schematic view of a further embodiment of a drug administration system;
Fig. 10B is a schematic graph showing data obtained by sensors of the embodiment of Fig. 10A;
Fig. 11A is a schematic view of a further embodiment of a drug administration system;
Fig. 11B is a schematic graph showing data obtained by sensors of the embodiment of Fig. 11A;
Fig. 12A is a schematic view of a further embodiment of a drug administration system;
Fig. 12B is a schematic graph showing data obtained by sensors of the embodiment of Fig. 12A;
Fig. 13A is a schematic view of a further embodiment of a drug administration system;
Fig. 13B is a schematic graph showing data obtained by sensors of the embodiment of Fig. 13A;
Fig. 14 is a schematic view of a further embodiment of a drug administration system;
Fig. 15 is a schematic view of a further embodiment of a drug administration system;
Fig. 16A is a schematic view of one embodiment of a drug administration device that includes a temperature sensor; and
Fig. 16B is an end view of the drug administration device of Fig. 16A.

### DETAILED DESCRIPTION

Certain exemplary embodiments will now be described to provide an overall understanding of the principles of the structure, function, manufacture, and use of the devices, systems, and methods disclosed herein. One or more examples of these embodiments are illustrated in the accompanying drawings. A person skilled in the art will understand that the devices, systems, and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments.

Further, in the present disclosure, like-named components of the embodiments generally have similar features, and thus within a particular embodiment each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. A person skilled in the art will appreciate that a dimension may not be a precise value but nevertheless be considered to be at about that value due to any number of factors such as manufacturing tolerances and sensitivity of measurement equipment. Sizes and shapes of the systems and devices, and the components thereof, can depend at least on the size and shape of components with which the systems and devices will be used.

Examples of various types of drug administration devices, namely: an autoinjector 100, an infusion pump 200, an inhaler 300, and a nasal spray device 400, are described below with reference to the hereinbefore referenced figures.

### Autoinjector

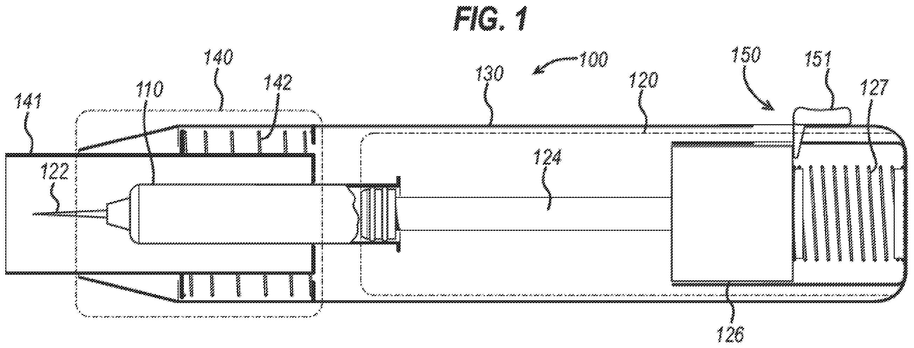

Fig. 1 is a schematic exemplary view of a first type of drug delivery device, namely an injection device, in this example an autoinjector 100, useable with embodiments described herein. The autoinjector 100 comprises a drug holder 110 which retains a drug to be dispensed and a dispensing mechanism 120 which is configured to dispense a drug from the drug holder 110 so that it can be administered to a patient. The drug holder 110 is typically in the form of a container which contains the drug, for example it may be provided in the form of a syringe or a vial, or be any other suitable container which can hold the drug. The autoinjector 100 comprises a discharge nozzle 122, for example a needle of a syringe, which is provided at a distal end of the drug holder 110. The dispensing mechanism 120 comprises a drive element 124, which itself may also comprise a piston and/or a piston rod, and drive mechanism 126. The dispensing mechanism 120 is located proximal to the end of the drug holder 110 and towards the proximal end of the autoinjector 100.

The autoinjector 100 comprises a housing 130 which contains the drug holder 110, drive element 124 and drive mechanism 126 within the body of the housing 130, as well as containing the discharge nozzle 122, which, prior to injection, would typically be contained fully within the housing, but which would extend out of the housing 130 during an injection sequence to deliver the drug. The dispensing mechanism 120 is arranged so that the drive element 124 is advanced through the drug holder 110 in order to dispense the drug through the discharge nozzle 122, thereby allowing the autoinjector to administer a drug retained in drug holder 110 to a patient. In some instances, a user may advance the drive element 124 through the drug holder 110 manually. In other instances, the drive mechanism 126 may include a stored energy source 127 which advances the drive element 124 without user assistance. The stored energy source 127 may include a resilient biasing member such as a spring, or a pressurized gas, or electronically powered motor and/or gearbox.

The autoinjector 100 includes a dispensing mechanism protection mechanism 140. The dispensing mechanism protection mechanism 140 typically has two functions. Firstly, the dispensing mechanism protection mechanism 140 can function to prevent access to the discharge nozzle 122 prior to and after injection. Secondly, the autoinjector 100 can function, such that when put into an activated state, e.g., the dispensing mechanism protection mechanism 140 is moved to an unlocked position, the dispensing mechanism 120 can be activated.

The protection mechanism 140 covers at least a part of the discharge nozzle 122 when the drug holder 110 is in its retracted position proximally within the housing 130. This is to impede contact between the discharge nozzle 122 and a user. Alternatively, or in addition, the protection mechanism 140 is itself configured to retract proximally to expose the discharge nozzle 122 so that it can be brought into contact with a patient. The protection mechanism 140 comprises a shield member 141 and return spring 142. Return spring 142 acts to extend the shield member 141 from the housing 130, thereby covering the discharge nozzle 122 when no force is applied to the distal end of the protection mechanism 140. If a user applies a force to the shield member 141 against the action of the return spring 142 to overcome the bias of the return spring 142, the shield member 141 retracts within the housing 130, thereby exposing the discharge nozzle 122. The protection mechanism 140 may alternatively, or in addition, comprise an extension mechanism (not shown) for extending the discharge nozzle 122 beyond the housing 130, and may further comprise a retracting mechanism (not shown) for retracting the discharge nozzle 122 within the housing 130. The protection mechanism 140 may alternatively, or in addition, comprise a housing cap and/or discharge nozzle boot, which can be attached to the autoinjector 100. Removal of the housing cap would typically also remove the discharge nozzle boot from the discharge nozzle 122.

The autoinjector 100 also includes a trigger 150. The trigger 150 comprises a trigger button 151 which is located on an external surface of the housing 130 so that it is accessible by a user of the autoinjector 100. When the trigger 150 is pressed by a user, it acts to release the drive mechanism 126 so that, via the drive element 124, the drug is then driven out of the drug holder 110 via the discharge nozzle 122.

The trigger 150 may also cooperate with the shield member 141 in such a way that the trigger 150 is prevented from being activated until the shield member 141 has been retracted proximally sufficiently into the housing 130 into an unlocked position, for example by pushing a distal end of the shield member 141 against the skin of a patient. When this has been done, the trigger 150 becomes unlocked, and the autoinjector 100 is activated such that the trigger 150 can be depressed and the injection and/or drug delivery sequence is then initiated. Alternatively, retraction of the shield member 141 alone in a proximal direction into the housing 130 can act to activate the drive mechanism 126 and initiate the injection and/or drug delivery sequence. In this way, the autoinjector 100 has device operation prevention mechanism which prevents dispensing of the drug by, for example, preventing accidental release of the dispensing mechanism 120 and/or accidental actuation of the trigger 150.

While the foregoing description relates to one example of an autoinjector, this example is presented purely for illustration, the present invention is not limited solely to such an autoinjector. A person skilled in the art understands that various modifications to the described autoinjector may be implemented within the scope of the present disclosure.

Autoinjectors of the present disclosure can be used to administer any of a variety of drugs, such as any of epinephrine, Rebif, Enbrel, Aranesp, atropine, pralidoxime chloride, and diazepam.

### Infusion Pump

**In** other circumstances, patients can require precise, continuous delivery of medication or medication delivery on a regular or frequent basis at set periodic intervals. Infusion pumps can provide such controlled drug infusion, by facilitating the administering of the drug at a precise rate that keeps the drug concentration within a therapeutic margin, without requiring frequent attention by a healthcare professional or the patient.

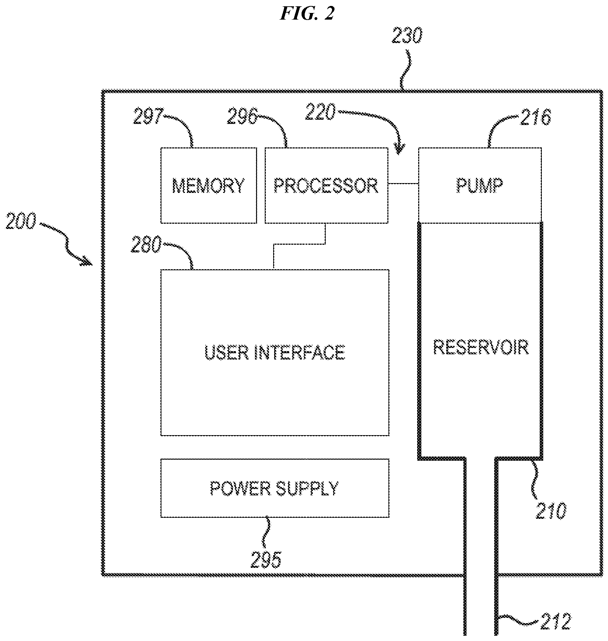

Fig. 2 is a schematic exemplary view of a second type of drug delivery device, namely an infusion pump 200, useable with the embodiments described herein. The infusion pump 200 comprises a drug holder 210 in the form of a reservoir for containing a drug to be delivered, and a dispensing mechanism 220 comprising a pump 216 adapted to dispense a drug contained in the reservoir, so that the drug can be delivered to a patient. These components of the infusion pump are located within housing 230. The dispensing mechanism 220 further comprises an infusion line 212. The drug is delivered from the reservoir upon actuation of the pump 216 via the infusion line 212, which may take the form of a cannula. The pump 216 may take the form of an elastomeric pump, a peristaltic pump, an osmotic pump, or a motor-controlled piston in a syringe. Typically, the drug is delivered intravenously, although subcutaneous, arterial and epidural infusions may also be used.

The infusion pumps of the present disclosure can be used to administer any of a variety of drugs, such as any of insulin, antropine sulfate, avibactam sodium, bendamustine hydrochloride, carboplatin, daptomycin, epinephrine, glucagon, levetiracetam, oxaliplatin, paclitaxel, pantoprazole sodium, treprostinil, vasopressin, voriconazole, and zoledronic acid.

The infusion pump 200 further comprises control circuitry, for example a processor 296 in addition to a memory 297 and a user interface 280, which together provide a triggering mechanism and/or dosage selector for the pump 200. The user interface 280 may be implemented by a display screen located on the housing 230 of the infusion pump 200. The control circuitry and user interface 280 can be located within the housing 230, or external thereto and communicate via a wired or wireless interface with the pump 216 to control its operation.

Actuation of the pump 216 is controlled by the processor 296 which is in communication with the pump 216 for controlling the pump's operation. The processor 296 may be programmed by a user (e.g., patient or healthcare professional), via a user interface 280. This enables the infusion pump 200 to deliver the drug to a patient in a controlled manner. The user can enter parameters, such as infusion duration and delivery rate. The delivery rate may be set by the user to a constant infusion rate or as set intervals for periodic delivery, typically within pre-programmed limits. The programmed parameters for controlling the pump 216 are stored in and retrieved from the memory 297 which is in communication with the processor 296. The user interface 280 may take the form of a touch screen or a keypad.

A power supply 295 provides power to the pump 216, and may take the form of an energy source which is integral to the pump 216 and/or a mechanism for connecting the pump 216 to an external source of power.

The infusion pump 200 may take on a variety of different physical forms depending on its designated use. It may be a stationary, non-portable device, e.g., for use at a patient's bedside, or it may be an ambulatory infusion pump which is designed to be portable or wearable. An integral power supply 295 is particularly beneficial for ambulatory infusion pumps.

While the foregoing description relates to one example of an infusion pump, this example is provided purely for illustration. The present disclosure is not limited to such an infusion pump. A person skilled in the art understands that various modifications to the described infusion pump may be implemented within the scope of the present disclosure. For example, the processor may be pre-programmed, such that it is not necessary for the infusion pump to include a user interface.

### Inhaler

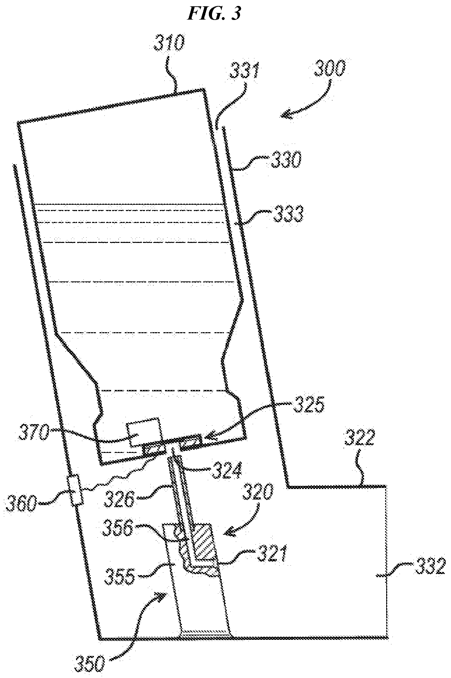

Fig. 3 is a schematic view of a third type of drug administration device, namely an inhaler 300. Inhaler 300 includes a drug holder 310 in the form of a canister. The drug holder 310 contains a drug that would typically be in solution or suspension with a suitable carrier liquid. The inhaler 300 further comprises a dispensing mechanism 320, which includes a pressurized gas for pressurizing the drug holder 310, a valve 325 and nozzle 321. The valve 325 forms an outlet of the drug holder 310. The valve 325 comprises a narrow opening 324 formed in the drug holder 310 and a movable element 326 that controls the opening 324. When the movable element 326 is in a resting position, the valve 325 is in a closed or unactuated state in which the opening 324 is closed and the drug holder 310 is sealed. When the movable element 326 is actuated from the resting position to an actuated position, the valve 325 is actuated into an open state in which the opening 324 is open. Actuation of the movable element 326 from the resting position to the actuated position comprises moving the movable element 326 into the drug holder 310. The movable element 326 is resiliently biased into the resting position. **In** the open state of the valve 325, the pressurized gas propels the drug in solution or suspension with the suitable liquid out of the drug holder 310 through the opening 324 at high speed. The high speed passage of the liquid through the narrow opening 324 causes the liquid to be atomized, that is, to transform from a bulk liquid into a mist of fine droplets of liquid and/or into a gas cloud. A patient may inhale the mist of fine droplets and/or the gas cloud into a respiratory passage. Hence, the inhaler 300 is capable of delivering a drug retained within the drug holder 310 into a respiratory passage of a patient.

The drug holder 310 is removably held within a housing 330 of the inhaler 300. A passage 333 formed in the housing 330 connects a first opening 331 in the housing 330 and a second opening 332 in the housing 330. The drug holder 310 is received within the passage 333. The drug holder 310 is slidably insertable through the first opening 331 of the housing 330 into the passage 333. The second opening 332 of the housing 330 forms a mouthpiece 322 configured to be placed in a patient's mouth or a nosepiece configured to be placed in a patient's nostril, or a mask configured to be placed over the patient's mouth and nose. The drug holder 310, the first opening 331 and the passage 333 are sized such that air can flow through the passage 333, around the drug holder 310, between the first opening 331 and the second opening 332. The inhaler 300 may be provided with a dispensing mechanism protection mechanism 140 in the form of a cap (not shown) which can be fitted to the mouthpiece 322.

Inhaler 300 further comprises a trigger 350 including a valve actuation feature 355 configured to actuate the valve 325 when the trigger 350 is activated. The valve actuation feature 355 is a projection of the housing 330 into the passage 333. The drug holder 310 is slidably movable within the passage 333 from a first position into a second position. **In** the first position, an end of the movable element 326 in the resting position abuts the valve actuation feature 355. **In** the second position, the drug holder 310 can be displaced towards the valve actuation feature 355 such that the valve actuation feature 355 moves the movable element 326 into the drug holder 310 to actuate the valve 325 into the open state. The user's hand provides the necessary force to move the drug holder 310 from the first position to the second position against the resiliently biased movable element 326. The valve actuation feature 355 includes an inlet 356, which is connected to the nozzle 321. The inlet 356 of the valve actuation feature 355 is sized and positioned to couple to the opening 324 of the valve 325 such that the ejected mist of droplets and/or gas cloud can enter the inlet 356 and exit from the nozzle 321 into the passage 333. The nozzle 321 assists in the atomization of the bulk liquid into the mist of droplets and/or gas cloud.

The valve 325 provides a metering mechanism 370. The metering mechanism 370 is configured to close the valve after a measured amount of liquid, and therefore, drug, has passed through the opening 324. This allows a controlled dose to be administered to the patient. Typically, the measured amount of liquid is pre-set, however, the inhaler 300 may be equipped with a dosage selector 360 that is user operable to change the defined amount of liquid.

While the foregoing description relates to one particular example of an inhaler, this example is purely illustrative. The description should not be seen as limited only to such an inhaler. A person skilled in the art understands that numerous other types of inhaler and nebulizers may be used with the present disclosure. For example, the drug may be in a powdered form, the drug may be in liquid form, or the drug may be atomized by other forms of dispensing mechanism 320 including ultrasonic vibration, compressed gas, a vibrating mesh, or a heat source.

The inhalers of the present disclosure can be used to administer any of a variety of drugs, such as any of mometasone, fluticasone, ciclesonide, budesonide, beclomethasone, vilanterol, salmeterol, formoterol, umeclidinium, glycopyrrolate, tiotropium, aclidinium, indacaterol, salmeterol, and olodaterol.

### Nasal Spray Device

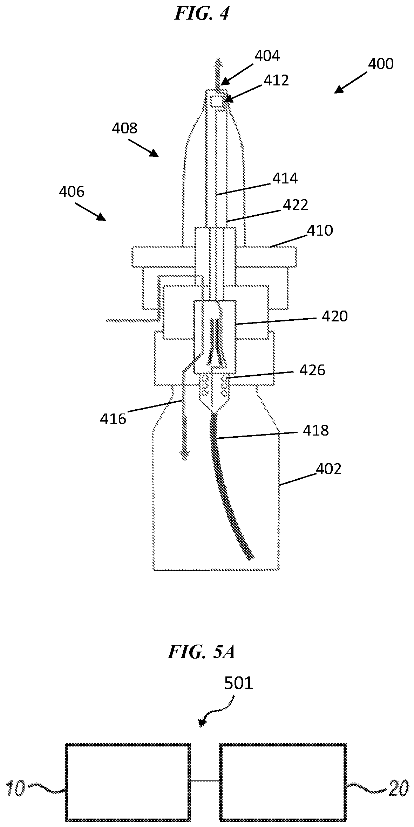

Fig. 4 is a schematic view of a fourth type of drug administration device, namely a nasal spray device 400. The nasal spray device 400 is configured to expel a drug into a nose of a patient. The nasal spray device 400 includes a drug holder 402 configured to contain a drug therein for delivery from the device 400 to a patient. The drug holder 102 can have a variety of configurations, such as a bottle reservoir, a cartridge, a vial (as in this illustrated embodiment), a blow-fill-seal (BFS) capsule, a blister pack, etc. In an exemplary embodiment, the drug holder 402 is a vial. An exemplary vial is formed of one or more materials, e.g., glass, polymer(s), etc. In some embodiments, a vial can be formed of glass. In other embodiments, a vial can be formed of one or more polymers. In yet other embodiments, different portions of a vial can be formed of different materials. An exemplary vial can include a variety of features to facilitate sealing and storing a drug therein, as described herein and illustrated in the drawings. However, a person skilled in the art will appreciate that the vials can include only some of these features and/or can include a variety of other features known in the art. The vials described herein are merely intended to represent certain exemplary embodiments.

An opening 404 of the nasal spray device 400 through which the drug exits the nasal spray device 400 is formed in in a dispensing head 406 of the nasal spray device 400 in a tip 408 of the dispensing head 406. The tip 408 is configured to be inserted into a nostril of a patient. In an exemplary embodiment, the tip 408 is configured to be inserted into a first nostril of the patient during a first stage of operation of the nasal spray device 400 and into a second nostril of the patient during a second stage of operation of the nasal spray device 400. The first and second stages of operation involve two separate actuations of the nasal spray device 400, a first actuation corresponding to a first dose of the drug being delivered and a second actuation corresponding to a second dose of the drug being delivered. In some embodiments, the nasal spray device 400 is configured to be actuated only once to deliver one nasal spray. In some embodiments, the nasal spray device 400 is configured to be actuated three or more times to deliver three or more nasal sprays, e.g., four, five, six, seven, eight, nine, ten, etc.

The dispensing head 406 includes a depth guide 410 configured to contact skin of the patient between the patient's first and second nostrils, such that a longitudinal axis of the dispensing head 406 is substantially aligned with a longitudinal axis of the nostril in which the tip 408 is inserted. A person skilled in the art will appreciate that the longitudinal axes may not be precisely aligned but nevertheless be considered to be substantially aligned due to any number of factors, such as manufacturing tolerances and sensitivity of measurement equipment.

In an exemplary embodiment, as in Fig. 4, the dispensing head 406 has a tapered shape in which the dispensing head 406 has a smaller diameter at its distal end than at its proximal end where the opening 404 is located. The opening 404 having a relatively small diameter facilitates spray of the drug out of the opening 404, as will be appreciated by a person skilled in the art. A spray chamber 412 through which the drug is configured to pass before exiting the opening 404 is located within a proximal portion of the tapered dispensing head 406, distal to the opening 404. When the drug passes through the spray chamber 412 at speed, the spray chamber 412 facilitates production of a fine mist that exits through the opening 404 with a consistent spray pattern. Arrow 414 in Fig. 4 illustrates a path of travel of the drug from the drug holder 402 and out of the opening 404.

In some embodiments, the dispensing head 406 can include two tips 408 each having an opening 404 therein such that the nasal spray device 400 is configured to simultaneously deliver doses of drug into two nostrils in response to a single actuation.

The dispensing head 406 is configured to be pushed toward the drug holder 402, e.g., depressed by a user pushing down on the depth guide 410, to actuate the nasal spray device 400. In other words, the dispensing head 406 is configured as an actuator to be actuated to drive the drug from the drug holder 402 and out of the nasal spray device 400. In an exemplary embodiment, the nasal spray device 400 is configured to be self-administered such that the user who actuates the nasal spray device 400 is the patient receiving the drug from the nasal spray device 400, although another person can actuate the nasal spray device 400 for delivery into another person.

The actuation, e.g., depressing, of the dispensing head 406 is configured to cause venting air to enter the drug holder 402, as shown by arrow 416 in Fig. 4. The air entering the drug holder 402 displaces drug in the drug holder through a tube 418 and then into a metering chamber 420, which displaces drug proximally through a cannula 422, through the spray chamber 412, and then out of the opening 404. In response to release of the dispensing head 406, e.g., a user stops pushing downward on the dispensing head 406, a bias spring 426 causes the dispensing head 406 to return to its default, resting position to position the dispensing head 406 relative to the drug holder 402 for a subsequent actuation and drug delivery.

While the foregoing description relates to one particular example of a nasal spray device, this example is purely illustrative. The description should not be seen as limited only to such a nasal spray device. A person skilled in the art understands that the nasal spray device 400 can include different features in different embodiments depending upon various requirements. For example, the nasal spray device 400 can lack the depth guide 410 and/or may include any one or more of a device indicator, a sensor, a communications interface, a processor, a memory, and a power supply.

The nasal spray devices of the present disclosure can be used to administer any of a variety of drugs, such as any of ketamine (e.g., Ketalar^{®}), esketamine (e.g., Spravato^{®}, Ketanest^{®}, and Ketanest-S^{®}), naloxone (e.g., Narcan^{®}), and sumatriptan (e.g., Imitrex^{®}).

### Drug Administration Device

As will be appreciated from the foregoing, various components of drug delivery devices are common to all such devices. These components form the essential components of a universal drug administration device. A drug administration device delivers a drug to a patient, where the drug is provided in a defined dosage form within the drug administration device.

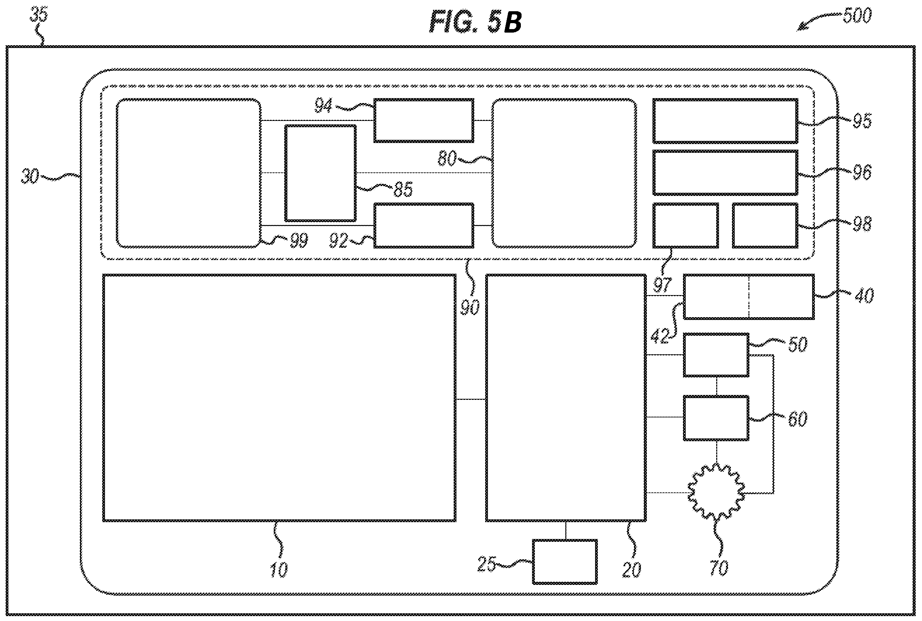

Fig. 5A is a generalized schematic view of such a universal drug administration device 501, and Fig. 5B is an exemplary embodiment of such a universal drug administration device 500. Examples of the universal drug administration device 500 include injection devices (e.g., autoinjectors, jet injectors, and infusion pumps), nasal spray devices, and inhalers.

As shown in Fig. 5A, drug administration device 501 includes in general form the features of a drug holder 10 and a dispensing mechanism 20. The drug holder 10 holds a drug in a dosage form to be administered. The dispensing mechanism 20 is configured to release the dosage form from the drug holder 10 so that the drug can be administered to a patient.

Fig. 5B shows a further universal drug administration device 500 which includes a number of additional features. A person skilled in the art understands that these additional features are optional for different embodiments, and can be utilized in a variety of different combinations such that the additional features may be present or may be omitted from a given embodiment of a particular drug administration device, depending upon requirements, such as the type of drug, dosage form of the drug, medical indication being treated with the drug, safety requirements, whether the device is powered, whether the device is portable, whether the device is used for self-administration, and many other requirements which will be appreciated by a person skilled in the art. Similar to the universal device of Fig. 5A, the drug administration device 500 comprises a housing 30 which accommodates the drug holder 10 and dispensing mechanism 20.

The device 500 is provided with a triggering mechanism 50 for initiating the release of the drug from the drug holder 10 by the dispensing mechanism 20. The device 500 includes the feature of a metering/dosing mechanism 70 which measures out a set dose to be released from the drug holder 10 via the dispensing mechanism 20. In this manner, the drug administration device 500 can provide a known dose of determined size. The device 500 comprises a dosage selector 60 which enables a user to set the dose volume of drug to be measured out by the metering mechanism 50. The dose volume can be set to one specific value of a plurality of predefined discrete dose volumes, or any value of predefined dose volume within a range of dose volumes.

The device 500 can comprise a device operation prevention mechanism 40 or 25 which when in a locked state will prevent and/or stop the dispensing mechanism 20 from releasing the drug out of the drug holder 10, and when in an unlocked state will permit the dispensing mechanism 20 to release the drug dosage from out of the drug holder 10. This can prevent accidental administration of the drug, for example to prevent dosing at an incorrect time, or for preventing inadvertent actuation. The device 500 also includes a dispensing mechanism protection mechanism 42 which prevents access to at least a part of the dispensing mechanism 20, for example for safety reasons. Device operation prevention mechanism 40 and dispensing mechanism protection mechanism 42 may be the same component.

The device 500 can include a device indicator 85 which is configured to present information about the status of the drug administration device and/or the drug contained therein. The device indicator 85 may be a visual indicator, such as a display screen, or an audio indicator. The device 500 includes a user interface 80 which can be configured to present a user of the device 500 with information about the device 500 and/or to enable the user to control the device 500. The device 500 includes a device sensor 92 which is configured to sense information relating to the drug administration device and/or the drug contained therein, for example dosage form and device parameters. As an example, in embodiments which include a metering mechanism 70 and a dosage selector 60, the embodiment may further include one or more device sensors 92 configured to sense one or more of: the dose selected by a user using dosage selector 60, the dose metered by the metering mechanism 70 and the dose dispensed by the dispensing mechanism 20. Similarly, an environment sensor 94 is provided which is configured to sense information relating to the environment in which the device 500 is present, such as the temperature of the environment, the humidity of the environment, location, and time. There may be a dedicated location sensor 98 which is configured to determine the geographical location of the device 500, e.g., via satellite position determination, such as GPS. The device 500 also includes a communications interface 99 which can communicate externally data which has been acquired from the various sensors about the device and/or drug.

If required, the device 500 comprises a power supply 95 for delivering electrical power to one or more electrical components of the device 500. The power supply 95 can be a source of power which is integral to device 500 and/or a mechanism for connecting device 500 to an external source of power. The drug administration device 500 also includes a device computer system 90 including processor 96 and memory 97 powered by the power supply 95 and in communication with each other, and optionally with other electrical and control components of the device 500, such as the environment sensor 94, location sensor 98, device sensor 92, communications interface 99, and/or indicator 85. The processor 96 is configured to obtain data acquired from the environment sensor 94, device sensor 92, communications interface 99, location sensor 98, and/or user interface 80 and process it to provide data output, for example to indicator 85 and/or to communications interface 99.

In some embodiments, the drug administration device 500 is enclosed in packaging 35. The packaging 35 may further include a combination of a processor 96, memory 97, user interface 80, device indicator 85, device sensor 92, location sensor 98 and/or environment sensors 94 as described herein, and these may be located externally on the housing of the device 500.

A person skilled in the art will appreciate that the universal drug administration device 500 comprising the drug holder 10 and dispensing mechanism 20 can be provided with a variety of the optional features described above, in a number of different combinations. Moreover, the drug administration device 500 can include more than one drug holder 10, optionally with more than one dispensing mechanism 20, such that each drug holder has its own associated dispensing mechanism 20.

### Drug Dosage Forms

Conventionally, drug administration devices utilize a liquid dosage form. It will be appreciated, however that other dosage forms are available.

One such common dosage form is a tablet. The tablet may be formed from a combination of the drug and an excipient that are compressed together. Other dosage forms are pastes, creams, powders, ear drops, and eye drops.

Further examples of drug dosage forms include dermal patches, drug eluting stents and intrauterine devices. In these examples, the body of the device comprises the drug and may be configured to allow the release of the drug under certain circumstances. For example, a dermal patch may comprise a polymeric composition containing the drug. The polymeric composition allows the drug to diffuse out of the polymeric composition and into the skin of the patient. Drug eluting stents and intrauterine devices can operate in an analogous manner. In this way, the patches, stents and intrauterine devices may themselves be considered drug holders with an associated dispensing mechanism.

Any of these dosage forms can be configured to have the drug release initiated by certain conditions. This can allow the drug to be released at a desired time or location after the dosage form has been introduced into the patient. In particular, the drug release may be initiated by an external stimulus. Moreover, these dosage forms can be contained prior to administration in a housing, which may be in the form of packaging. This housing may contain some of the optional features described above which are utilized with the universal drug administration device 500.

The drug administered by the drug administration devices of the present disclosure can be any substance that causes a change in an organism's physiology or psychology when consumed. Examples of drugs that the drug administration devices of the present disclosure can administer include 5-alpha-reductase inhibitors, 5-aminosalicylates, 5HT3 receptor antagonists, ACE inhibitors with calcium channel blocking agents, ACE inhibitors with thiazides, adamantane antivirals, adrenal cortical steroids, adrenal corticosteroid inhibitors, adrenergic bronchodilators, agents for hypertensive emergencies, agents for pulmonary hypertension, aldosterone receptor antagonists, alkylating agents, allergenics, alpha-glucosidase inhibitors, alternative medicines, amebicides, aminoglycosides, aminopenicillins, aminosalicylates, AMPA receptor antagonists, amylin analogs, analgesic combinations, analgesics, androgens and anabolic steroids, Angiotensin Converting Enzyme Inhibitors, angiotensin II inhibitors with calcium channel blockers, angiotensin II inhibitors with thiazides, angiotensin receptor blockers, angiotensin receptor blockers and neprilysin inhibitors, anorectal preparations, anorexiants, antacids, anthelmintics, anti-angiogenic ophthalmic agents, anti-CTLA-4 monoclonal antibodies, anti-infectives, anti-PD-1 monoclonal antibodies, antiadrenergic agents (central) with thiazides, antiadrenergic agents (peripheral) with thiazides, antiadrenergic agents, centrally acting, antiadrenergic agents, peripherally acting, antiandrogens, antianginal agents, antiarrhythmic agents, antiasthmatic combinations, antibiotics/antineoplastics, anticholinergic antiemetics, anticholinergic antiparkinson agents, anticholinergic bronchodilators, anticholinergic chronotropic agents, anticholinergics/antispasmodics, anticoagulant reversal agents, anticoagulants, anticonvulsants, antidepressants, antidiabetic agents, antidiabetic combinations, antidiarrheals, antidiuretic hormones, antidotes, antiemetic/antivertigo agents, antifungals, antigonadotropic agents, antigout agents, antihistamines, antihyperlipidemic agents, antihyperlipidemic combinations, antihypertensive combinations, antihyperuricemic agents, antimalarial agents, antimalarial combinations, antimalarial quinolones, antimanic agents, antimetabolites, antimigraine agents, antineoplastic combinations, antineoplastic detoxifying agents, antineoplastic interferons, antineoplastics, antiparkinson agents, antiplatelet agents, antipseudomonal penicillins, antipsoriatics, antipsychotics, antirheumatics, antiseptic and germicides, antithyroid agents, antitoxins and antivenins, antituberculosis agents, antituberculosis combinations, antitussives, antiviral agents, antiviral boosters, antiviral combinations, antiviral interferons, anxiolytics, sedatives, and hypnotics, aromatase inhibitors, atypical antipsychotics, azole antifungals, bacterial vaccines, barbiturate anticonvulsants, barbiturates, BCR-ABL tyrosine kinase inhibitors, benzodiazepine anticonvulsants, benzodiazepines, beta blockers with calcium channel blockers, beta blockers with thiazides, beta-adrenergic blocking agents, beta-lactamase inhibitors, bile acid sequestrants, biologicals, bisphosphonates, bone morphogenetic proteins, bone resorption inhibitors, bronchodilator combinations, bronchodilators, calcimimetics, calcineurin inhibitors, calcitonin, calcium channel blocking agents, carbamate anticonvulsants, carbapenems, carbapenems/beta-lactamase inhibitors, carbonic anhydrase inhibitor anticonvulsants, carbonic anhydrase inhibitors, cardiac stressing agents, cardioselective beta blockers, cardiovascular agents, catecholamines, cation exchange resins, CD20 monoclonal antibodies, CD30 monoclonal antibodies, CD33 monoclonal antibodies, CD38 monoclonal antibodies, CD52 monoclonal antibodies, CDK 4/6 inhibitors, central nervous system agents, cephalosporins, cephalosporins/beta-lactamase inhibitors, cerumenolytics, CFTR combinations, CFTR potentiators, CGRP inhibitors, chelating agents, chemokine receptor antagonist, chloride channel activators, cholesterol absorption inhibitors, cholinergic agonists, cholinergic muscle stimulants, cholinesterase inhibitors, CNS stimulants, coagulation modifiers, colony stimulating factors, contraceptives, corticotropin, coumarins and indandiones, cox-2 inhibitors, decongestants, dermatological agents, diagnostic radiopharmaceuticals, diarylquinolines, dibenzazepine anticonvulsants, digestive enzymes, dipeptidyl peptidase 4 inhibitors, diuretics, dopaminergic antiparkinsonism agents, drugs used in alcohol dependence, echinocandins, EGFR inhibitors, estrogen receptor antagonists, estrogens, expectorants, factor Xa inhibitors, fatty acid derivative anticonvulsants, fibric acid derivatives, first generation cephalosporins, fourth generation cephalosporins, functional bowel disorder agents, gallstone solubilizing agents, gamma-aminobutyric acid analogs, gamma-aminobutyric acid reuptake inhibitors, gastrointestinal agents, general anesthetics, genitourinary tract agents, GI stimulants, glucocorticoids, glucose elevating agents, glycopeptide antibiotics, glycoprotein platelet inhibitors, glycylcyclines, gonadotropin releasing hormones, gonadotropin-releasing hormone antagonists, gonadotropins, group I antiarrhythmics, group II antiarrhythmics, group III antiarrhythmics, group IV antiarrhythmics, group V antiarrhythmics, growth hormone receptor blockers, growth hormones, guanylate cyclase-C agonists, H. pylori eradication agents, H2 antagonists, hedgehog pathway inhibitors, hematopoietic stem cell mobilizer, heparin antagonists, heparins, HER2 inhibitors, herbal products, histone deacetylase inhibitors, hormones, hormones/antineoplastics, hydantoin anticonvulsants, hydrazide derivatives, illicit (street) drugs, immune globulins, immunologic agents, immunostimulants, immunosuppressive agents, impotence agents, in vivo diagnostic biologicals, incretin mimetics, inhaled anti-infectives, inhaled corticosteroids, inotropic agents, insulin, insulin-like growth factors, integrase strand transfer inhibitor, interferons, interleukin inhibitors, interleukins, intravenous nutritional products, iodinated contrast media, ionic iodinated contrast media, iron products, ketolides, laxatives, leprostatics, leukotriene modifiers, lincomycin derivatives, local injectable anesthetics, local injectable anesthetics with corticosteroids, loop diuretics, lung surfactants, lymphatic staining agents, lysosomal enzymes, macrolide derivatives, macrolides, magnetic resonance imaging contrast media, mast cell stabilizers, medical gas, meglitinides, metabolic agents, methylxanthines, mineralocorticoids, minerals and electrolytes, miscellaneous agents, miscellaneous analgesics, miscellaneous antibiotics, miscellaneous anticonvulsants, miscellaneous antidepressants, miscellaneous antidiabetic agents, miscellaneous antiemetics, miscellaneous antifungals, miscellaneous antihyperlipidemic agents, miscellaneous antihypertensive combinations, miscellaneous antimalarials, miscellaneous antineoplastics, miscellaneous antiparkinson agents, miscellaneous antipsychotic agents, miscellaneous antituberculosis agents, miscellaneous antivirals, miscellaneous anxiolytics, sedatives and hypnotics, miscellaneous bone resorption inhibitors, miscellaneous cardiovascular agents, miscellaneous central nervous system agents, miscellaneous coagulation modifiers, miscellaneous diagnostic dyes, miscellaneous diuretics, miscellaneous genitourinary tract agents, miscellaneous GI agents, miscellaneous hormones, miscellaneous metabolic agents, miscellaneous ophthalmic agents, miscellaneous otic agents, miscellaneous respiratory agents, miscellaneous sex hormones, miscellaneous topical agents, miscellaneous uncategorized agents, miscellaneous vaginal agents, mitotic inhibitors, monoamine oxidase inhibitors, mouth and throat products, mTOR inhibitors, mucolytics, multikinase inhibitors, muscle relaxants, mydriatics, narcotic analgesic combinations, narcotic analgesics, nasal anti-infectives, nasal antihistamines and decongestants, nasal lubricants and irrigations, nasal preparations, nasal steroids, natural penicillins, neprilysin inhibitors, neuraminidase inhibitors, neuromuscular blocking agents, neuronal potassium channel openers, next generation cephalosporins, nicotinic acid derivatives, NK1 receptor antagonists, NNRTIs, non-cardioselective beta blockers, non-iodinated contrast media, non-ionic iodinated contrast media, non-sulfonylureas, Nonsteroidal anti-inflammatory drugs, NS5A inhibitors, nucleoside reverse transcriptase inhibitors (NRTIs), nutraceutical products, nutritional products, ophthalmic anesthetics, ophthalmic anti-infectives, ophthalmic anti-inflammatory agents, ophthalmic antihistamines and decongestants, ophthalmic diagnostic agents, ophthalmic glaucoma agents, ophthalmic lubricants and irrigations, ophthalmic preparations, ophthalmic steroids, ophthalmic steroids with anti-infectives, ophthalmic surgical agents, oral nutritional supplements, other immunostimulants, other immunosuppressants, otic anesthetics, otic anti-infectives, otic preparations, otic steroids, otic steroids with anti-infectives, oxazolidinedione anticonvulsants, oxazolidinone antibiotics, parathyroid hormone and analogs, PARP inhibitors, PCSK9 inhibitors, penicillinase resistant penicillins, penicillins, peripheral opioid receptor antagonists, peripheral opioid receptor mixed agonists/antagonists, peripheral vasodilators, peripherally acting antiobesity agents, phenothiazine antiemetics, phenothiazine antipsychotics, phenylpiperazine antidepressants, phosphate binders, PI3K inhibitors, plasma expanders, platelet aggregation inhibitors, platelet-stimulating agents, polyenes, potassium sparing diuretics with thiazides, potassium-sparing diuretics, probiotics, progesterone receptor modulators, progestins, prolactin inhibitors, prostaglandin D2 antagonists, protease inhibitors, protease-activated receptor-1 antagonists, proteasome inhibitors, proton pump inhibitors, psoralens, psychotherapeutic agents, psychotherapeutic combinations, purine nucleosides, pyrrolidine anticonvulsants, quinolones, radiocontrast agents, radiologic adjuncts, radiologic agents, radiologic conjugating agents, radiopharmaceuticals, recombinant human erythropoietins, renin inhibitors, respiratory agents, respiratory inhalant products, rifamycin derivatives, salicylates, sclerosing agents, second generation cephalosporins, selective estrogen receptor modulators, selective immunosuppressants, selective phosphodiesterase-4 inhibitors, selective serotonin reuptake inhibitors, serotonin-norepinephrine reuptake inhibitors, serotoninergic neuroenteric modulators, sex hormone combinations, sex hormones, SGLT-2 inhibitors, skeletal muscle relaxant combinations, skeletal muscle relaxants, smoking cessation agents, somatostatin and somatostatin analogs, spermicides, statins, sterile irrigating solutions, streptogramins, streptomyces derivatives, succinimide anticonvulsants, sulfonamides, sulfonylureas, synthetic ovulation stimulants, tetracyclic antidepressants, tetracyclines, therapeutic radiopharmaceuticals, therapeutic vaccines, thiazide diuretics, thiazolidinediones, thioxanthenes, third generation cephalosporins, thrombin inhibitors, thrombolytics, thyroid drugs, TNF alfa inhibitors, tocolytic agents, topical acne agents, topical agents, topical allergy diagnostic agents, topical anesthetics, topical anti-infectives, topical anti-rosacea agents, topical antibiotics, topical antifungals, topical antihistamines, topical antineoplastics, topical antipsoriatics, topical antivirals, topical astringents, topical debriding agents, topical depigmenting agents, topical emollients, topical keratolytics, topical non-steroidal anti-inflammatories, topical photochemotherapeutics, topical rubefacient, topical steroids, topical steroids with anti-infectives, transthyretin stabilizers, triazine anticonvulsants, tricyclic antidepressants, trifunctional monoclonal antibodies, ultrasound contrast media, upper respiratory combinations, urea anticonvulsants, urea cycle disorder agents, urinary anti-infectives, urinary antispasmodics, urinary pH modifiers, uterotonic agents, vaccine combinations, vaginal anti-infectives, vaginal preparations, vasodilators, vasopressin antagonists, vasopressors, VEGF/VEGFR inhibitors, viral vaccines, viscosupplementation agents, vitamin and mineral combinations, vitamins, or VMAT2 inhibitors. The drug administration devices of the present disclosure may administer a drug selected from epinephrine, Rebif, Enbrel, Aranesp, atropine, pralidoxime chloride, diazepam, insulin, antropine sulfate, avibactam sodium, bendamustine hydrochloride, carboplatin, daptomycin, epinephrine, levetiracetam, oxaliplatin, paclitaxel, pantoprazole sodium, treprostinil, vasopressin, voriconazole, zoledronic acid, mometasone, fluticasone, ciclesonide, budesonide, beclomethasone, vilanterol, salmeterol, formoterol, umeclidinium, glycopyrrolate, tiotropium, aclidinium, indacaterol, salmeterol, and olodaterol.

As mentioned above, any of a variety of drugs can be delivered using a drug administration device. Examples of drugs that can be delivered using a drug administration device as described herein include Remicade^{®} (infliximab), Stelara^{®} (ustekinumab), Simponi^{®} (golimumab), Simponi Aria^{®} (golimumab), Darzalex^{®} (daratumumab), Tremfya^{®} (guselkumab), Eprex^{®} (epoetin alfa), Risperdal Constra^{®} (risperidone), Invega Sustenna^{®} (paliperidone palmitate), Spravato^{®} (esketamine), ketamine, and Invega Trinza^{®} (paliperidone palmitate).

### Drug Housing

As described above, a dosage form can be provided in a holder that is appropriate for the particular dosage form being utilized. For example, a drug in a liquid dosage form can be held prior to administration within a holder in the form of a vial with a stopper, or a syringe with a plunger. A drug in solid or powder dosage form, e.g., as tablets, may be contained in a housing which is arranged to hold the tablets securely prior to administration.

The housing may comprise one or a plurality of drug holders, where each holder contains a dosage form, e.g., the drug can be in a tablet dosage form and the housing can be in the form of a blister pack, where a tablet is held within each of a plurality of holders. The holders being in the form of recesses in the blister pack.

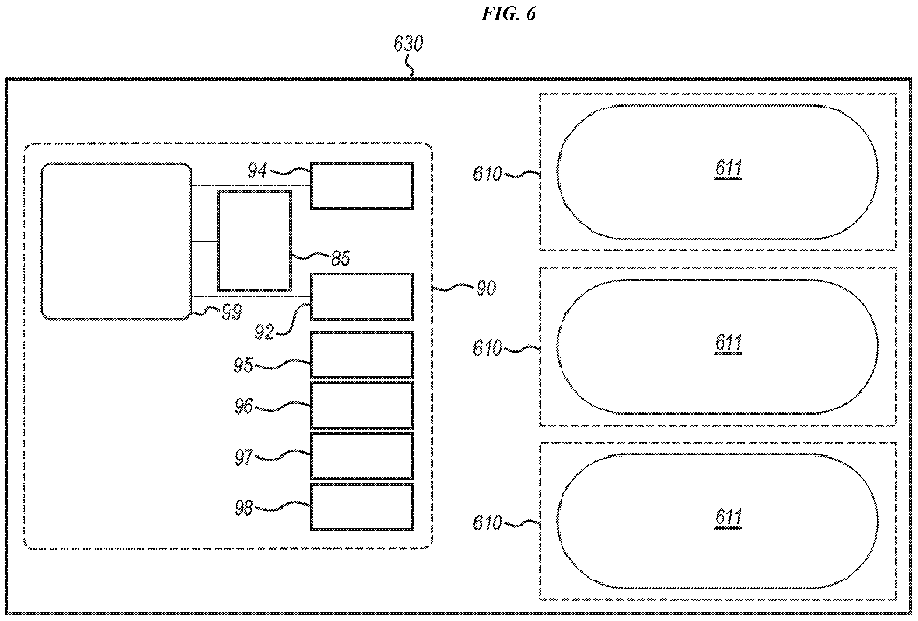

Fig. 6 depicts a housing 630 that comprises a plurality of drug holders 610 that each contain a dosage form 611. The housing 630 may have at least one environment sensor 94, which is configured to sense information relating to the environment in which the housing 630 is present, such as the temperature of the environment, time or location. The housing 630 may include at least one device sensor 92, which is configured to sense information relating to the drug of the dosage form 611 contained within the holder 610. There may be a dedicated location sensor 98 which is configured to determine the geographical location of the housing 630, e.g., via satellite position determination, such as GPS.

The housing 630 may include an indicator 85 which is configured to present information about the status of the drug of the dosage form 611 contained within the holder 610 to a user of the drug housing. The housing 630 may also include a communications interface 99 which can communicate information externally via a wired or wireless transfer of data pertaining to the drug housing 630, environment, time or location and/or the drug itself.

If required, the housing 630 may comprise a power supply 95 for delivering electrical power to one or more electrical components of the housing 630. The power supply 95 can be a source of power which is integral to housing 630 and/or a mechanism for connecting the housing 630 to an external source of power. The housing 630 may also include a device computer system 90 including processor 96 and memory 97 powered by the power supply 95 and in communication with each other, and optionally with other electrical and control components of the housing 630, such as the environment sensor 94, location sensor 98, device sensor 92, communications interface 99, and/or indicator 85. The processor 96 is configured to obtain data acquired from the environment sensor 94, device sensor 92, communications interface 99, location sensor 98, and/or user interface 80 and process it to provide data output, for example to indicator 85 and/or to communications interface 99.

The housing 630 can be in the form of packaging. Alternatively, additional packaging may be present to contain and surround the housing 630.

The holder 610 or the additional packaging may themselves comprise one or more of the device sensor 92, the environment sensor 94, the indicator 85, the communications interface 99, the power supply 95, location sensor 98, and device computer system including the processor 96 and the memory 85, as described above.

### Electronic Communication

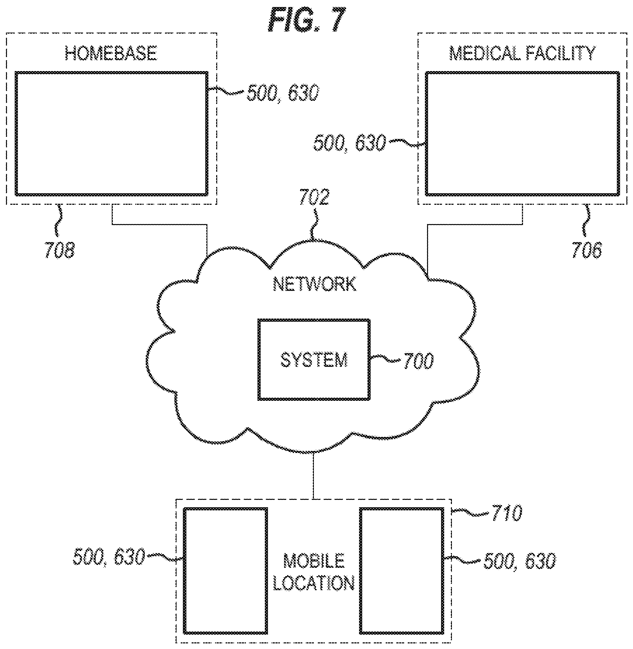

As mentioned above, communications interface 99 may be associated with the drug administration device 500 or drug housing 630, by being included within or on the housing 30, 630, or alternatively within or on the packaging 35. Such a communications interface 99 can be configured to communicate with a remote computer system, such as central computer system 700 shown in Fig. 7. As shown in Fig. 7, the communications interface 99 associated with drug administration device 500 or housing 630 is configured to communicate with a central computer system 700 through a communications network 702 from any number of locations such as a medical facility 706, e.g., a hospital or other medical care center, a home base 708 (e.g., a patient's home or office or a care taker's home or office), or a mobile location 710. The communications interface 99 can be configured to access the system 700 through a wired and/or wireless connection to the network 702. In an exemplary embodiment, the communications interface 99 of Fig. 6 is configured to access the system 700 wirelessly, e.g., through Wi-Fi connection(s), which can facilitate accessibility of the system 700 from almost any location in the world.

A person skilled in the art will appreciate that the system 700 can include security features such that the aspects of the system 700 available to any particular user can be determined based on, e.g., the identity of the user and/or the location from which the user is accessing the system. To that end, each user can have a unique username, password, biometric data, and/or other security credentials to facilitate access to the system 700. The received security parameter information can be checked against a database of authorized users to determine whether the user is authorized and to what extent the user is permitted to interact with the system, view information stored in the system, and so forth.

### Computer System

As discussed herein, one or more aspects or features of the subject matter described herein, for example components of the central computer system 700, processor 96, power supply 95, memory 97, communications interface 99, user interface 80, device indicators 85, device sensors 92, environment sensors 94 and location sensors 98, can be realized in digital electronic circuitry, integrated circuitry, specially designed application specific integrated circuits (ASICs), field programmable gate arrays (FPGAs) computer hardware, firmware, software, and/or combinations thereof. These various aspects or features can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device. The programmable system or computer system may include clients and servers. A client and server are generally remote from each other and typically interact through a communications network, e.g., the Internet, a wireless wide area network, a local area network, a wide area network, or a wired network. The relationship of client and server arises by virtue of computer programs running on the respective computers and having a client-server relationship to each other.

The computer programs, which can also be referred to as programs, software, software applications, applications, components, or code, include machine instructions for a programmable processor, and can be implemented in a high-level procedural language, an object-oriented programming language, a functional programming language, a logical programming language, and/or in assembly/machine language. As used herein, the term "machine-readable medium" refers to any computer program product, apparatus and/or device, such as for example magnetic discs, optical disks, memory, and Programmable Logic Devices (PLDs), used to provide machine instructions and/or data to a programmable processor, including a machine-readable medium that receives machine instructions as a machine-readable signal. The term "machine-readable signal" refers to any signal used to provide machine instructions and/or data to a programmable processor. The machine-readable medium can store such machine instructions non-transitorily, such as for example as would a non-transient solid-state memory or a magnetic hard drive or any equivalent storage medium. The machine-readable medium can alternatively or additionally store such machine instructions in a transient manner, such as for example as would a processor cache or other random access memory associated with one or more physical processor cores.

To provide for interaction with a user, one or more aspects or features of the subject matter described herein, for example user interface 80 (which can be integrated or separate to the administration device 500 or housing 630), can be implemented on a computer having a display screen, such as for example a cathode ray tube (CRT) or a liquid crystal display (LCD) or a light emitting diode (LED) monitor for displaying information to the user. The display screen can allow input thereto directly (e.g., as a touch screen) or indirectly (e.g., via an input device such as a keypad or voice recognition hardware and software). Other kinds of devices can be used to provide for interaction with a user as well. For example, feedback provided to the user can be any form of sensory feedback, such as for example visual feedback, auditory feedback, or tactile feedback; and input from the user may be received in any form, including, but not limited to, acoustic, speech, or tactile input. As described above, this feedback may be provided via one or more device indicators 85 in addition to the user interface 80. The device indicators 85 can interact with one or more of device sensor(s) 92, environment sensor(s) 94 and/or location sensor(s) 98 in order to provide this feedback, or to receive input from the user.

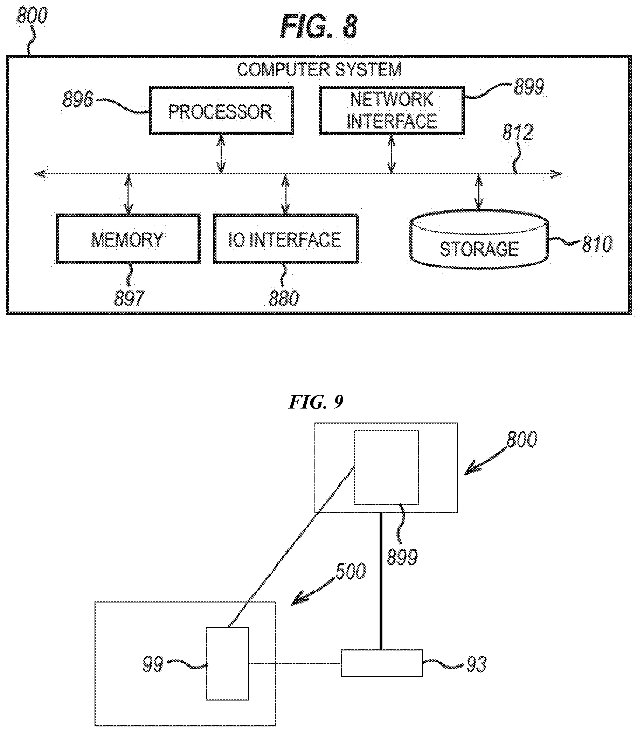

Fig. 8 illustrates one exemplary embodiment of the computer system 700, depicted as computer system 800. The computer system includes one or more processors 896 configured to control the operation of the computer system 800. The processor(s) 896 can include any type of microprocessor or central processing unit (CPU), including programmable general-purpose or special-purpose microprocessors and/or any one of a variety of proprietary or commercially available single or multi-processor systems. The computer system 800 also includes one or more memories 897 configured to provide temporary storage for code to be executed by the processor(s) 896 or for data acquired from one or more users, storage devices, and/or databases. The memory 897 can include read-only memory (ROM), flash memory, one or more varieties of random access memory (RAM) (e.g., static RAM (SRAM), dynamic RAM (DRAM), or synchronous DRAM (SDRAM)), and/or a combination of memory technologies.

The various elements of the computer system are coupled to a bus system 812. The illustrated bus system 812 is an abstraction that represents any one or more separate physical busses, communication lines/interfaces, and/or multi-drop or point-to-point connections, connected by appropriate bridges, adapters, and/or controllers. The computer system 800 also includes one or more network interface(s) 899 (also referred to herein as a communications interface), one or more input/output (IO) interface(s) 880, and one or more storage device(s) 810.

The communications interface(s) 899 are configured to enable the computer system to communicate with remote devices, e.g., other computer systems and/or devices 500 or housings 630, over a network, and can be, for example, remote desktop connection interfaces, Ethernet adapters, and/or other local area network (LAN) adapters. The IO interface(s) 880 include one or more interface components to connect the computer system 800 with other electronic equipment. For example, the IO interface(s) 880 can include high speed data ports, such as universal serial bus (USB) ports, 1394 ports, Wi-Fi, Bluetooth, etc. Additionally, the computer system can be accessible to a human user, and thus the IO interface(s) 880 can include displays, speakers, keyboards, pointing devices, and/or various other video, audio, or alphanumeric interfaces. The storage device(s) 810 include any conventional medium for storing data in a nonvolatile and/or non-transient manner. The storage device(s) 810 are thus configured to hold data and/or instructions in a persistent state in which the value(s) are retained despite interruption of power to the computer system. The storage device(s) 810 can include one or more hard disk drives, flash drives, USB drives, optical drives, various media cards, diskettes, compact discs, and/or any combination thereof and can be directly connected to the computer system or remotely connected thereto, such as over a network. In an exemplary embodiment, the storage device(s) 810 include a tangible or non-transitory computer readable medium configured to store data, e.g., a hard disk drive, a flash drive, a USB drive, an optical drive, a media card, a diskette, or a compact disc.

The elements illustrated in Fig. 8 can be some or all of the elements of a single physical machine. In addition, not all of the illustrated elements need to be located on or in the same physical machine.

The computer system 800 can include a web browser for retrieving web pages or other markup language streams, presenting those pages and/or streams (visually, aurally, or otherwise), executing scripts, controls and other code on those pages/streams, accepting user input with respect to those pages/streams (e.g., for purposes of completing input fields), issuing HyperText Transfer Protocol (HTTP) requests with respect to those pages/streams or otherwise (e.g., for submitting to a server information from the completed input fields), and so forth. The web pages or other markup language can be in HyperText Markup Language (HTML) or other conventional forms, including embedded Extensible Markup Language (XML), scripts, controls, and so forth. The computer system 800 can also include a web server for generating and/or delivering the web pages to client computer systems.

As shown in Fig. 7, the computer system 800 of Fig. 8 as described above may form the components of the central computer system 700 which is in communication with one or more of the device computer systems 90 of the one or more individual drug administration devices 500 or housings 630. Data, such as operational data of the devices 500 or housings 630, medical data acquired of patients by such devices 500 or housings 630 can be exchanged between the central and device computer systems 700, 90.

As mentioned the computer system 800 as described above may also form the components of a device computer system 90 which is integrated into or in close proximity to the drug administration device 500 or housing 630. In this regard, the one or more processors 896 correspond to the processor 96, the network interface 799 corresponds to the communications interface 99, the IO interface 880 corresponds to the user interface 80, and the memory 897 corresponds to the memory 97. Moreover, the additional storage 810 may also be present in device computer system 90.

In an exemplary embodiment, the computer system 800 can form the device computer system 90 as a single unit, e.g., contained within a single drug administration device housing 30, contained within a single package 35 for one or more drug administration devices 500, or a housing 630 that comprises a plurality of drug holders 610. The computer system 800 can form the central computer system 700 as a single unit, as a single server, or as a single tower.

The single unit can be modular such that various aspects thereof can be swapped in and out as needed for, e.g., upgrade, replacement, maintenance, etc., without interrupting functionality of any other aspects of the system. The single unit can thus also be scalable with the ability to be added to as additional modules and/or additional functionality of existing modules are desired and/or improved upon.

The computer system can also include any of a variety of other software and/or hardware components, including by way of example, operating systems and database management systems. Although an exemplary computer system is depicted and described herein, it will be appreciated that this is for sake of generality and convenience. In other embodiments, the computer system may differ in architecture and operation from that shown and described here. For non-limiting examples, the memory 897 and storage device 810 can be integrated together or the communications interface 899 can be omitted if communication with another computer system is not necessary.

### Implementations

It can be desirable to monitor compliance with the guidance that is associated with the drugs that are administered to a patient in various dosage forms. This can provide assurance that correct procedures are being followed and avoid the adoption of incorrect and potentially dangerous approaches. Further, this can also enable optimization of the administration of the drug to the patient.

Further, it can be desirable to provide a drug administration system which does not require a patient to monitor their response to drug dosing schemes themselves and then adjust their drug dosing scheme. Furthermore, it can be desirable to provide a drug administration system which takes account of factors which affect the patient's response to the drug dosing scheme but which change over time.

Fig. 9 shows an exemplary embodiment of a drug administration system that includes a drug administration device 500 (Figs. 5B and 7) and a computer system 800 (Fig. 8). Specific examples of the drug administration devices 500 are discussed herein, such as the autoinjector 100 (Fig. 1), the infusion pump 200 (Fig. 2), and the inhaler 300 (Fig. 3), with other types possible such as a nasal spray device. The drug administration device 500 includes the communications interface 99 configured to communicate with the communications interface 899 of the computer system 800. The drug administration system further includes at least one sensor 93 configured to sense a parameter, and thereby obtain sensor data, and communicate the sensor data to the drug administration device 500. The drug administration device 500 is configured to utilize a first drug dosing scheme when a drug is delivered to a patient from the drug administration device 500 and to determine a second drug dosing scheme for delivering the drug to the patient from the drug administration device 500. The second drug dosing scheme is dependent on dosing data from the computer system 800 and on the sensor data from the at least one sensor 93. The sensor data can be obtained by the at least one sensor 93 during and/or after the delivery of the drug according to the first drug dosing scheme. The drug administration device 500 is also configured to utilize the second drug dosing scheme when the drug is delivered to the patient from the drug administration device 500 after the first drug dosing scheme has been utilized to deliver the drug to the patient from the drug administration device 500.

The sensor data can relate to factor(s) that affect a patient's response to the first drug dosing scheme. In this way, the drug administration system may better optimize a patient's dosing scheme by taking into account this data without a patient having to monitor and process the data themselves, which may be time consuming, confusing, and/or impossible due to inability to precisely measure the factor(s).

The first drug dosing scheme can be dependent on dosing data communicated to the drug administration device 500 from the computer system 800. This communication enables the first drug dosing scheme to be dictated via the computer system 800. The drug administration device 500 can thus be configured to determine the first drug dosing scheme for delivering the drug to the patient before utilizing the first drug dosing scheme when the drug is delivered to the patient. Alternatively, the first dosing scheme can be a default dosing scheme contained on the drug administration device 500, e.g., stored in the memory 97 thereof. The first dosing scheme can be present on the drug administration device 500 from the time of manufacturing the drug administration device 500 or at least at a time before a user (e.g., the patient, the patient's care giver, or a medical professional) receives the drug administration device 500 for use with the patient.

The dosing data can relate to data that indicates drug dosing parameter(s) to be utilized by the drug administration device 500 when delivering the drug therefrom to the patient. In this way, the dosing data can be used as a basis for determining the first drug dosing scheme. The drug dosing scheme is a collection of one or more drug dosing parameters associated with the delivery of drug. Examples of drug dosing parameters include at least one of drug delivery rate, drug delivery volume, drug delivery duration, drug delivery frequency, and timing of drug delivery. As mentioned, each of the first and second drug dosing schemes is made up of one or more of such drug dosing parameters.

Each of the first and second dosing schemes is an algorithm stored in the memory 97 of the drug administration device 500 that is executable on board the drug administration device 500 by the processor 96. The algorithm is stored in the form of one or more sets of pluralities of data points defining and/or representing instructions, notifications, signals, etc. to control functions of the device 500 and administration of the drug from the device 500. As discussed herein, data received by the drug administration device 500, e.g., as pluralities of data points via the communications interface 99 thereof, can be used, e.g., by the processor 96, to change at least one drug dosing parameter of the algorithm. The at least one drug dosing parameter is among the algorithm's data points and are thus each able to be changed by changing one or more of the stored pluralities of data points of the algorithm.

The data received by the drug administration device 500, e.g., from the computer system 800 and from the at least one sensor 93, can identify the drug dosing parameter(s) to change and each parameter's updated value, or the data received by the drug administration device 500 can include input parameter(s) that the drug administration device 500 is configured to use in executing a calculation (e.g., in execution of a calculation algorithm stored in the memory 97) to determine the correct value for each of the drug dosing parameter(s) in the algorithm for the drug dosing scheme. The calculation algorithm is stored in the form of one or more sets of pluralities of data points. The calculation algorithm utilized by the drug administration device 500 can be already present in the memory 97 prior to receipt of the dosing data. Alternatively, the calculation algorithm utilized by the drug administration device 500 can be provided as part of the dosing data that also includes the input parameter(s).

The input parameter(s) that are utilized as part of the drug dosing parameter determination that involves executing the calculation algorithm can include clinical data such as patient age, patient weight, urine pH of the patient, patient temperature, a condition of the patient which is being treated, and the existence of other disease states. The input parameters can include data relating to activity or toxicity of the drug, such as a minimum therapeutic dose, a maximum therapeutic dose, and side effects. The input parameters can include pharmacokinetic data such as absorption rate, distribution rate, metabolism, and excretion rate of the particular drug to be delivered to the patient. The drug dosing algorithm can take these parameters as inputs and calculate a drug dosing scheme.

After the at least one drug dosing parameter has been changed, subsequent execution of the algorithm administers another dose of the drug according to the algorithm, which reflects the current drug dosing scheme whether the first drug dosing scheme or the second drug dosing scheme (or a drug dosing scheme subsequent to the second drug dosing scheme, as the drug dosing scheme can be changed any number of times to help optimize drug delivery over time). Alternatively, each changed algorithm, e.g., each different drug dosing scheme, can be stored in the memory 97 with the processor 96 executing the most recent of the stored drug dosing schemes to cause drug delivery from the drug administration device 500. Storing each of the drug dosing schemes may require a larger memory 97 than if only one drug dosing scheme is stored at a time, but storing each of the drug dosing schemes simultaneously in the memory 97 facilitate analysis of drug delivery, patient compliance, and/or of the patient's treatment.

By allowing for changes in the drug dosing scheme over time, drug delivery over time can be managed for the patient, e.g., by a medical professional providing input for the drug delivery change to the system 700, to increase the beneficial results of the drug. Changing the at least one drug dosing parameter is automated to improve patient outcomes. Thus, the drug administration system can be configured to facilitate personalized medicine based on the patient to provide a smart system for drug delivery.

As discussed above, the dosing data can contain a direct indication of the drug dosing parameter(s) to be utilized by the drug administration device 500, or, alternatively, the dosing data can contain input parameter(s) that are used by the drug administration device 500 as part of a determination calculation that is carried out on the drug administration device 500. Either approach enables the drug administration device 500 to modify the current drug dosing scheme to improve the effectiveness of the drug dosing scheme. Similarly, the sensor data can contain a direct indication of the drug dosing parameter(s) to be utilized by the drug administration device 500, or, alternatively, the sensor data can contain input parameter(s) that are used by the drug administration device 500 as part of a determination calculation that is carried out on the drug administration device 500.

As noted herein, the drug administration device 500 is also configured to determine a second drug dosing scheme. The second drug dosing scheme is calculated based on a plurality of inputs as discussed above, e.g., based on the dosing data from the computer system 800 and/or the sensor data from the at least one sensor 93. The plurality of inputs can also include user input data that is input to the drug administration device 500 via the user interface 80. The plurality of inputs can be utilized to adjust the second drug dosing scheme relative to the first drug dosing scheme and may thereby assist in optimizing the drug dosing scheme for the patient.

The computer system 800 can be configured to determine the second drug dosing scheme similar to that discussed above regarding the drug administration device's use of the algorithm to determine the second drug dosing scheme, and the computer system 800 can be configured to send the second drug dosing scheme to the drug administration device 500. In this way, the drug administration device 500 can have a smaller and/or less expensive memory 97 and/or processor 96 since the drug administration device 500 would need to be configured to store and execute the second drug dosing scheme without also needing to receive the sensor data or perform any calculations to determine the second drug dosing scheme (other than recognizing that the second drug dosing scheme has replaced the first drug dosing scheme as the current drug dosing scheme). To facilitate the computer system's determination of the second drug dosing scheme, the sensor data obtained by the at least one sensor 93 during and/or after the delivery of the drug according to the first drug dosing scheme can be communicated from the at least one sensor 93 to the computer system 800, e.g., using communications interfaces thereof. The computer system 800 also has the dosing data that can be used in determining the second drug dosing scheme.

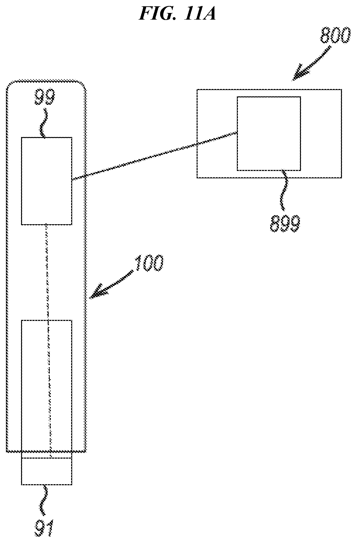

The at least one sensor 93 can include one or more different types of sensors and can include one or more of a same type of sensor. Examples of sensors that can be used in any combination as the at least one sensor 93 include a patient monitoring sensor 91 (Fig. 11A) configured to obtain sensor data relating to the condition of the patient, the device sensor 92 (Fig. 5B) configured to obtain sensor data relating to the drug administration device 500 and/or the drug contained therein, the environment sensor 94 (Fig. 5B) configured to obtain sensor data relating to the environment in which the drug administration device 500 is present, and the location sensor 98 (Fig. 5B) configured to obtain sensor data relating to geographical location of the drug administration device 500. The at least one sensor 93 can be part of the drug administration device 500 or, as shown in this illustrated embodiment of Fig. 9, the at least one sensor 93 can be a distinct element from the drug administration device 500. When the at least one sensor 93 includes a plurality of sensors, one or more of the sensors can be on board the drug administration device 500 and/or one or more of the sensors can be a distinct element from the drug administration device 500.

The patient monitoring sensor 91 is configured to obtain sensor data relating to a condition of the patient. Sensor data relating to the condition of the patient can include any one or more of blood oxygen level of the patient (e.g., using a blood oxygen sensor), blood glucose level of the patient (e.g., using a glucose monitor, etc.), blood pressure of the patient (e.g., using a blood pressure monitor, etc.), heart rate of the patient (e.g., using a heart rate monitor, etc.), temperature of the patient, metabolic rate of the patient, anaphylactic response of the patient, a length and/or severity of an exacerbation of asthma symptoms, time taken for a physiological response to occur in response to a drug administration, magnitude of patient physiological response to a drug administration, tissue thickness, tissue density, and patient body weight. Impedance sensors, for example, are a type of patient sensor which can be utilized to sense tissue thickness and tissue density.

The device sensor 92 is configured to obtain sensor data relating to the drug administration device 500 and/or the drug contained therein, which in an exemplary embodiment can include one or more of orientation of the drug administration device 500 (e.g., using a gyro, an accelerometer, a tilt/angle switch (mercury free), a position sensor, etc.) and a temperature of the drug in the drug administration device 500 (e.g., using a temperature sensor such as a thermistor, a thermocoupler, a thermistor, etc.), as orientation and temperature may have particular relevance for a drug dosing scheme.

The environment sensor 94 is configured to obtain sensor data relating to the environment in which the drug administration device 500 is present, which in an exemplary embodiment can include a sensor configured to collect one or more of sensor data relating to air quality of the environment in which the device 500 is present (e.g., using a UV sensor, etc.), pollen count of the environment in which the device 500 is present (e.g., using a pollen sensor, etc.), humidity of an environment in which the device 500 is present (e.g., using a thermistor, a humistor, a hygrometer, etc.), and temperature in which the device 500 is present (e.g., using a temperature sensor such as a thermistor, a thermocoupler, a thermistor, etc.). Measures of air quality include measures of nitrogen dioxide, ground-level ozone, carbon monoxide, and particulates.

The drug administration device 500 can be configured to determine a first drug dosing scheme for delivering a first drug to the patient and to determine a second drug dosing scheme for delivering a second drug to the patient, where the second drug is different to the first drug. The drug administration device 500 can be configured to deliver both of the first and second drugs to the patient. For example, the first drug can be a drug for intermittent reliever therapy, and the second drug can be a drug for regular preventer therapy. Drugs suitable for these uses are known by a person skilled in the art. The drug administration device 500 can be configured to adjust each of the first and second drug dosing schemes as discussed above so that delivery of each of the first and second drugs may be optimized for the patient.

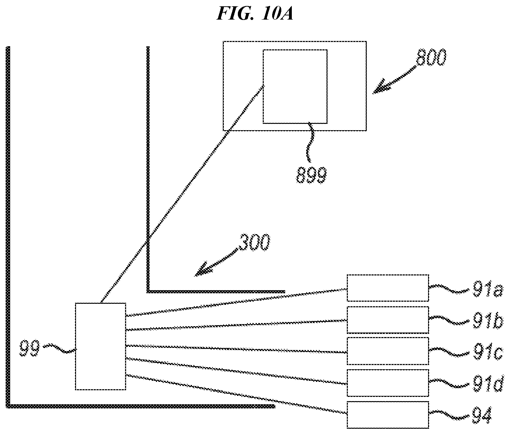

Fig. 10A is a schematic diagram of an exemplary embodiment of the drug administration system of Fig. 9. In the embodiment shown in Fig. 10A, the drug administration device 500 is an inhaler 300 (Fig. 3). The sensor 91 (Fig. 9) in this illustrated embodiment includes a first patient monitoring sensor 91a configured to obtain sensor data relating to a blood oxygen level of the patient, a second patient monitoring sensor 91b configured to obtain data relating to blood pressure (BP) of the patient, a third patient monitoring sensor 91c configured to obtain data relating patient heart rate, an environment sensor 94 (Fig. 5B) configured to obtain data relating to air quality and pollen count of the environment in which the inhaler 300 is present, and a fourth patient monitoring sensor 91d configured to obtain sensor data relating to body weight (body mass) of the patient. In use, the sensors 91a, 91b, 91c, 91d, 94 obtain data and communicate the data to the inhaler 300 as discussed above.

Accordingly, as discussed above with respect to Fig. 9, the inhaler 300 of the embodiment of Fig. 10A is configured to utilize a first drug dosing scheme to deliver a drug therefrom to the patient. The inhaler 300 is configured to then determine a second drug dosing scheme for delivering the drug therefrom to the patient with the second drug dosing scheme being dependent on dosing data from the computer system 800 and on sensor data from the patient monitoring sensors 91a, 91b, 91c, 91d and the environment sensor 94 that is obtained by the sensors 91a, 91b, 91c, 91d, 94 during and/or after the delivery of the drug according to the first drug dosing scheme. The inhaler 300 is also configured to utilize the second drug dosing scheme when the drug is delivered to the patient at some point after the delivery according to the first dosing scheme and after the determination of the second drug dosing scheme.

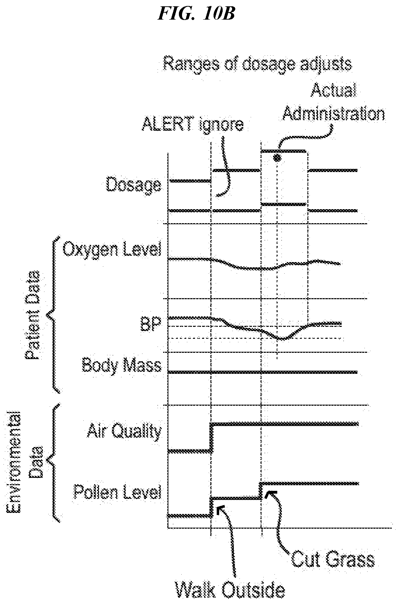

Fig. 10B shows a schematic graph of an example of data obtained by the first patient monitoring sensor 91a, the second patient monitoring sensor 91b, the environment sensor 94, and the fourth body monitoring sensor 91d in addition to three drug dosing scheme adjustments that occur at times indicated by vertical dotted lines. A first one of the drug dosing scheme adjustments occurs in response to changes that occur when the patient goes outside, wearing or otherwise with the sensors 91a, 91b, 91c, 91d, 94 (e.g., as part of a smart watch being worn by the patient, as part of an implanted device, as part of a sensor adhered to a skin surface of the patient, as part of the inhaler 300 being carried by the patient, etc.), and experiences changes in pollen level and air quality. For this first one of the drug dosing scheme adjustments, the inhaler 300 is configured to adjust the drug dosing scheme dependent on the sensor data which indicates increased pollen level and decreased air quality via an increased measure of particulates, for example, as well as a magnitude of the patient's response to the increased pollen level and air quality as indicated via the measurements of blood oxygen level (which decreases) and blood pressure (which also decreases). Thus, the adjusted drug dosing scheme specifies an increased size of drug dose in comparison to that of the first drug dosing scheme prior to the patient walking outside in order to better manage the patient's symptoms. No drug dose is delivered to the patient using either of the first two drug dosing schemes.

A second one of the drug dosing scheme adjustments occurs in response to changes that occur when the patient, wearing or otherwise with the sensors 91a, 91b, 91c, 91d, 94, is cutting grass outside (e.g., mowing a lawn). For this second one of the drug dosing scheme adjustments, the inhaler 300 is configured to adjust the drug dosing scheme dependent on the sensor data which indicates increased pollen level, as well as a magnitude of the patient's response to the increased pollen level and reflects the activity of grass cutting as indicated via the measurements of blood oxygen level (which increases) and blood pressure (which decreases). Thus, the adjusted drug dosing scheme specifies an increased size of drug dose in comparison to that of the immediately prior drug dosing scheme in order to better manage the patient's symptoms. A drug dose is delivered to the patient in accordance with this drug dosing scheme, as shown by the "Actual Administration" noted on the graph of Fig. 10B.

A third one of the drug dosing scheme adjustments occurs in response to changes that occur at some point after the drug dose ("Actual Administration" noted on the graph of Fig. 10B) has been delivered to the patient. For this third one of the drug dosing scheme adjustments, the inhaler 300 is configured to adjust the drug dosing scheme dependent on the sensor data which reflects the drug administration as indicated via the measurements of blood oxygen level (which increases) and blood pressure (which increases). Thus, the adjusted drug dosing scheme specifies a reduced size of drug dose in comparison to that of the immediately prior drug dosing scheme in order to better manage the patient's symptoms. No drug dose is delivered to the patient using this adjusted drug dosing scheme. The drug dosing scheme can be adjusted any number of times after this third drug dosing scheme adjustment to continue better managing the patient's symptoms.

Optionally, as discussed above with respect to Fig. 9, the inhaler 300 can be configured to determine the initial drug dosing scheme in Fig. 10B prior to the patient walking outside, with the initial drug dosing scheme being dependent on dosing data.

In the embodiments of Figs. 11A to 13B, patient comfort and efficacy of a drug dosing scheme may be improved by enabling a drug administration device to adjust factors of one or more of a discharge nozzle advance depth of the drug administration device's discharge nozzle during delivery of a drug to the patient from the drug administration device, a discharge nozzle velocity of the discharge nozzle during delivery of the drug to the patient from the drug administration device, and a discharge nozzle acceleration of the discharge nozzle during delivery of the drug to the patient from the drug administration device.

The discharge nozzle advance depth during delivery of the drug is a maximum depth to which the discharge nozzle is configured to extend into the patient during the entire drug delivery event, from when the discharge nozzle first extends into a patient to when the discharge nozzle exits. For an autoinjector (e.g., the autoinjector 100 of Fig. 1), a discharge nozzle advance depth of the autoinjector's discharge nozzle during delivery of a drug to a patient from the autoinjector can be in a range of 2 mm to 25 mm below the patient's skin surface.

The discharge nozzle velocity during delivery of the drug is a maximum velocity at which the discharge nozzle is configured to travel when the discharge nozzle extends into the patient during the entire drug delivery event, from when the discharge nozzle first extends into the patient to when the discharge nozzle exits. For an autoinjector (e.g., the autoinjector 100 of Fig. 1), a discharge nozzle velocity of the autoinjector's discharge nozzle during delivery of a drug to a patient from the autoinjector can be in a range of 0.01 m/s to 0.2 m/s.

The discharge nozzle acceleration during delivery of the drug is a maximum acceleration at which the discharge nozzle is configured to accelerate during the entire drug delivery event, from when the discharge nozzle first extends into the patient to when the discharge nozzle exits. For an autoinjector (e.g., the autoinjector 100 of Fig. 1), a discharge nozzle velocity of the autoinjector's discharge nozzle during delivery of a drug to a patient from the autoinjector can be in a range of 0.002 m/s² to 0.2 m/s².

Fig. 11A is a schematic diagram of an exemplary embodiment of a drug administration system that includes the computer system 800 (Figs. 8 and 9), the drug administration device 500 (Figs. 5B, 7, and 9), which in this embodiment includes the autoinjector 100 (Fig. 1), and the at least one patient monitoring sensor 91, such as an impedance sensor configured to detect density and/or thickness of tissue of a patient. The drug administration system is generally configured and used similar to that discussed above regarding the drug administration system of Fig. 9. The sensor 91 can be positioned, for example, on a part of the autoinjector 100 which comes into direct contact with an injection site as is shown in Fig. 11A. The first and second drug dosing schemes of this embodiment specify at least drug delivery volume and drug delivery rate, e.g., the drug dosing parameters of the drug dosing scheme that can be changed in the algorithm that defines the drug dosing scheme are drug delivery volume and drug delivery rate.

Hence, in the embodiment of Fig. 11A, the autoinjector 100 is configured to utilize the first drug dosing scheme when the drug is delivered to the patient, determine a second drug dosing scheme dependent on dosing data from the computer system 800 and on sensor data from the at least one patient monitoring sensor 91 that includes data relating to the density and/or thickness of the tissue of the patient. Tissue density can influence pain experienced by the patient during the injection. Therefore, a slower rate of delivery of an injection can be utilized in order to reduce the pain of the injection when denser tissue is detected. Tissue can be denser due to scarring, for example, or because the tissue is muscle tissue rather than subcutaneous tissue.

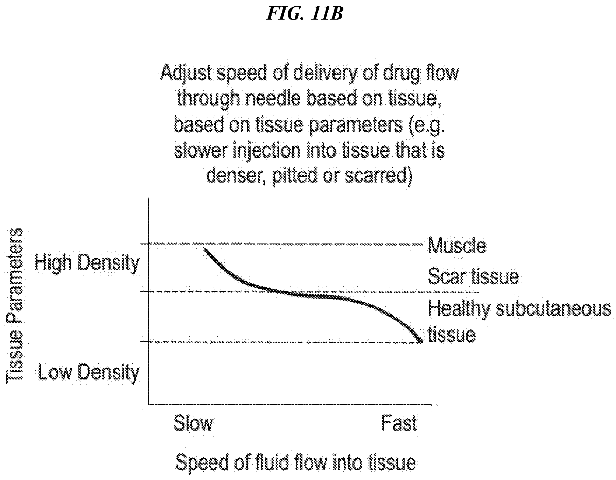

Fig. 11B shows a schematic graph of an example of tissue density data obtained by the at least one patient monitoring sensor 91 in addition to drug delivery speed (also referred to herein as "drug delivery rate"). The first dosing scheme can be dependent on dosing data. This dosing data can be based not on the particular patient or on the particular injection site of the patient but instead on an injection into a tissue of a predetermined density which corresponds to a density of unscarred tissue. If the at least one patient monitoring sensor 91 obtains data indicating that the tissue of the patient has a density higher than the predetermined density, the autoinjector 100 can be configured to determine a second dosing scheme which specifies a lower drug delivery rate than that of the first dosing scheme. In this way, the second drug dosing scheme may improve the comfort of the patient during drug delivery.

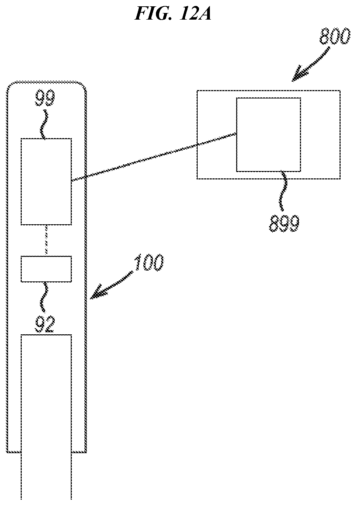

Fig. 12A is a schematic diagram of an exemplary embodiment of a drug administration system that includes the computer system 800 (Figs. 8 and 9), the drug administration device 500 (Figs. 5B, 7, and 9), which in this embodiment includes the autoinjector 100 (Fig. 1), and the at least one sensor 93 (Fig. 9), which in this illustrated embodiment includes at least one device sensor 92 (Fig. 5B) configured to obtain sensor data relating to at least a temperature of a drug contained in the autoinjector 100. The drug administration system is generally configured and used similar to that discussed above regarding the drug administration system of Fig. 9. The first and second drug dosing schemes of this embodiment specify at least drug delivery volume and drug delivery rate. Accordingly, the autoinjector 100 of the embodiment of Fig. 12A is configured to utilize a first drug dosing scheme when the drug is delivered to the patient, determine a second drug dosing scheme dependent on dosing data from the computer system 800 and on sensor data from the at least one device sensor 92. If the drug has been refrigerated, injecting the drug slowly will improve patient comfort if the drug has not had sufficient time to warm to room temperature before injection.

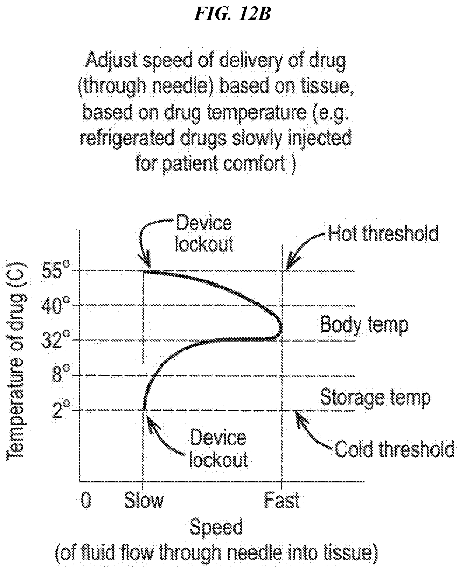

Fig. 12B shows a schematic diagram of an example of temperature data obtained by the at least one device sensor 92 of the embodiment of Fig. 12A in addition to the drug delivery speed. The autoinjector 100 is configured to determine a first dosing scheme dependent on the dosing data communicated from the computer system 800, with the dosing data being based on injection of a drug at a predetermined temperature. However, if the at least one device sensor 92 obtains data indicating that the drug is at a temperature cooler than the predetermined temperature, the autoinjector 100 is configured to determine a second dosing scheme which specifies a lower drug delivery rate than that of the first dosing scheme to improve patient comfort.

Further, the autoinjector 100 is configured to operate the device operation prevention mechanism 40 (Fig. 5B), e.g., the dispensing mechanism protection mechanism 140 (Fig. 1), to prevent the autoinjector 100 from being operated before the drug is delivered according to either of the first and second dosing schemes if the at least one device sensor 92 detects that the temperature of the drug is above a predetermined high threshold ("Hot threshold" in Fig. 12B) and/or below a predetermined low threshold ("Cold threshold in Fig 12B) which are considered painful and/or dangerous to a patient. Examples of such thresholds are an upper temperature limit (predetermined high threshold) of 55 degrees Celsius and a lower temperature limit (predetermined low threshold) of 2 degrees Celsius.

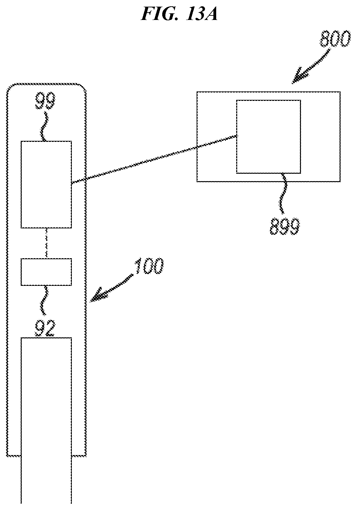

Fig. 13A is a schematic diagram of another exemplary embodiment of a drug administration system that includes the computer system 800 (Figs. 8 and 9), the drug administration device 500 (Figs. 5B, 7, and 9), which in this embodiment includes the autoinjector 100 (Fig. 1), and the at least one device sensor 92 (Fig. 5B), which in this illustrated embodiment is configured to obtain sensor data relating to the temperature of a drug contained in the autoinjector 100. The drug administration system is generally configured and used similar to that discussed above regarding the drug administration system of Fig. 9. The first and second drug dosing schemes of this embodiment specify at least drug delivery volume and needle advance depth of the autoinjector's needle (e.g., discharge nozzle 122) during delivery of the drug to the patient. Accordingly, the autoinjector 100 of the embodiment of Fig. 13A is configured to utilize a first drug dosing scheme when the drug is delivered to the patient, determine a second drug dosing scheme dependent on dosing data from the computer system 800 and on sensor data from the at least one device sensor 92. The dosing data on which the first drug dosing scheme is dependent is based on delivering the drug at a predetermined temperature. If the drug contained in the autoinjector 100 is sensed to be cooler than the predetermined temperature and more viscous, advancing the needle to a shorter needle advance depth into the patient will help ensure patient comfort. Thus, in this embodiment, if the sensor data relating to the temperature of the drug indicates a temperature lower than that used to determine the first drug dosing scheme, the drug administration device 100 is configured to specify a needle penetration depth in the drug dosing scheme (e.g., in the algorithm that defines the drug dosing scheme) which is shorter than that specified by the first dosing scheme.

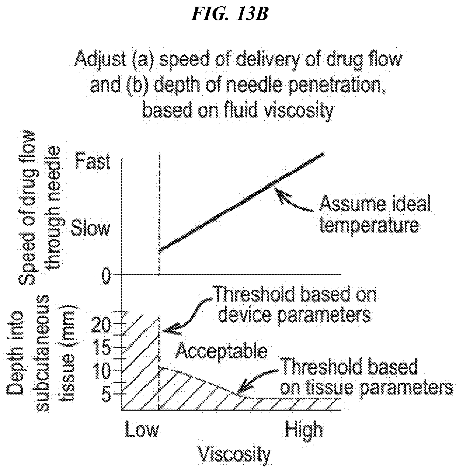

Fig. 13B shows a schematic graph of an embodiment of sensor data obtained in the embodiments of Figs. 12A and 13A, in which the sensor data in the illustrated graph represents viscosity of the drug contained in the autoinjector 100. The embodiments of Figs. 12A and 13A can be combined so that the drug dosing scheme specifies both drug delivery rate (Fig. 12A embodiment) and needle advance depth (Fig. 13A embodiment) and so that the drug administration device 500 (the autoinjector 100) is configured to determine a second drug dosing scheme specifying both drug delivery rate and needle penetration depth.

In another example, the drug administration device 500 (the autoinjector 100) and the at least one device sensor 92 can include an orientation sensor. Thus, if the orientation sensor indicates that the drug administration device 500 is positioned such that the drug delivery rate may be reduced, for example if the device 500 is upside down, the drug administration device 500 can be configured to determine a second drug dosing scheme which takes account of this likely reduced delivery rate, e.g., by increasing a speed at which the drug delivery occurs to account for the sensed orientation being indicative of a reduced drug delivery rate.

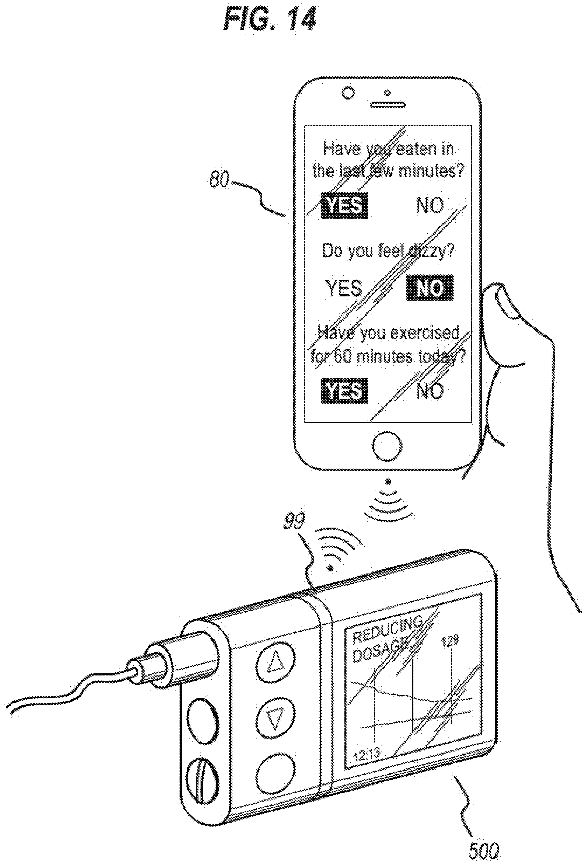

Fig. 14 shows a schematic diagram of an exemplary embodiment of a drug administration system that includes the computer system 800 (Figs. 8 and 9), which in this illustrated embodiment is a smart phone, and the drug administration device 500, which includes a communications interface 99 configured to communicate with the communications interface 899 of the computer system 800. The drug administration system is generally configured and used similar to that discussed above regarding the drug administration system of Fig. 9. The computer system 800 includes at least one user interface 80 configured to obtain user input data and communicate the user input data to the drug administration device 500. The drug administration device 500 is configured to utilize a first drug dosing scheme when a drug is delivered to a patient from the drug administration device 500, determine a second drug dosing scheme for delivering the drug to the patient that is dependent on dosing data from the computer system 800 and on user input data from the at least one user interface 80 that is obtained by the user interface 80 during and/or after the delivery of the drug according to the first drug dosing scheme, and utilize the second drug dosing scheme when the drug is delivered to the patient at some point after the delivery according to the first dosing scheme and after the determination of the second drug dosing scheme.

The user input data can relate to factors that affect the patient's response to the first drug dosing scheme and/or the patient's perceived response to the first drug dosing scheme. **In** this way, the system may better optimize the current drug dosing scheme by taking into account the patient's response and/or perceived response to drug delivery even if it is not possible to use sensors to obtain the patient's response and/or perceived response to drug delivery. For example, a patient planning to eat a large meal may input user data into the user interface 80 since eating is a factor that may affect the patient's response to the first drug dosing scheme. The computer system 800 is configured to send the user input data to the drug administration device 500. The device 500 is configured to use the user input data in determining the second drug dosing scheme to administer the drug, e.g., a fast-acting insulin, immediately before the patient eats the large meal and thereby enact a second drug dosing scheme that takes account of this inputted factor. In another example, if the patient experiences discomfort when the drug administration device 500 delivers the drug according to the first drug dosing scheme, the user can input this feedback of their perceived response to the first drug dosing scheme using the user interface 80, the computer system 800 can send the user input data to the drug administration device 500, and the drug administration device can then determine the second drug dosing scheme dependent on this user input data by, for example, reducing drug delivery rate, discharge nozzle advance depth, discharge nozzle velocity, and/or discharge nozzle acceleration. Thus the second drug dosing scheme can be adjusted with an intention of improving the patient's perceived response to drug delivery.

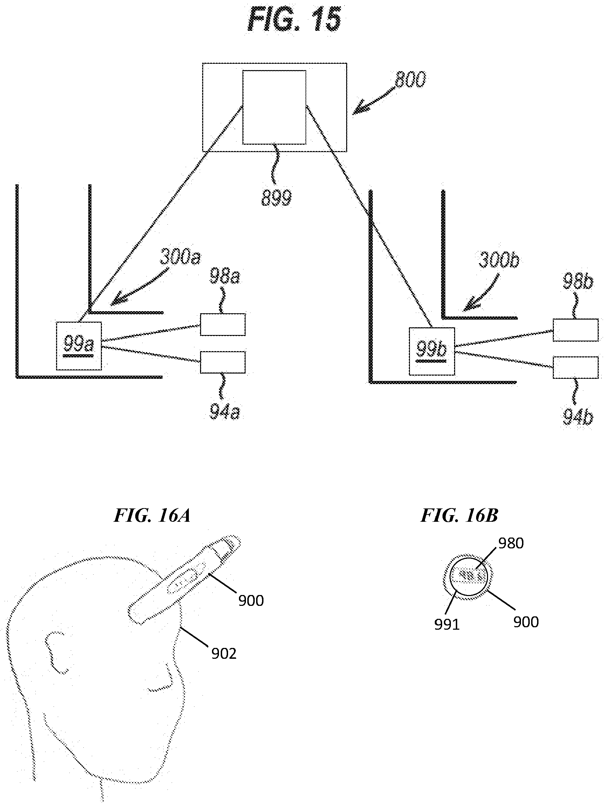

Fig. 15 shows a schematic diagram of an exemplary embodiment of a drug administration system that includes the computer system 800 (Figs. 8 and 9), first and second drug administration devices 300a, 300b each configured to communicate with the computer system 800, at least one sensor 94a, 98a configured to obtain sensor data and communicate the sensor data to the first drug administration device 300a, and at least one sensor 94b, 98b configured to obtain supplementary sensor data and communicate the supplementary sensor data to the second drug administration device 300b. The drug administration system is generally configured and used similar to that discussed above regarding the drug administration system of Fig. 9 except that instead of one drug administration device, the drug administration system of Fig. 15 includes a plurality of drug administration devices 300a, 300b. The drug administration system of Fig. 15 includes two drug administration device 300a, 300b, but a drug administration system can include any plural number of drug administration devices and otherwise be configured and used similar to the system of Fig. 15.

The second drug administration device 300b is configured to communicate the supplementary sensor data obtained by the at least one sensor 94b, 98b to the computer system 800 (e.g., using a communications interface 99b of the second drug administration device 300b and the communications interface 899 of the computer system 800), and the computer system 800 is configured to communicate the supplementary sensor data received from the second drug administration device 300b to the first drug administration device 300a (e.g., using a communications interface 99a of the first drug administration device 300a and the communications interface 899 of the computer system 800). The first drug administration device 300a is configured to utilize the supplementary sensor data received from the computer system 800 as part of its determination of a second dosing scheme. Alternatively, the computer system 800 can be configured to utilize the supplementary sensor data received from the second drug administration device 300b to calculate the adjustment required for the second dosing scheme for delivery of a drug contained in the first drug administration device 300a to a patient. This adjustment can then be communicated from the computer system 800 to the first drug administration device 300a.

The use of the second drug administration device 300b (and any additional drug administration devices) in the drug administration system enables the first drug administration device 300a (or the computer system 800, in embodiments in which the computer system 800 determines and sends the second dosing scheme to the first drug administration device 300a) to determine the second drug dosing scheme dependent on not just dosing data from the computer system 800 and/or on sensing data from its at least one sensor 94a, 98a but additionally on supplementary sensor data relating to the second drug administration device 300b when determining the second drug dosing scheme for delivery of the drug contained in the first drug administration device 300a to a patient.

The second drug administration device 300b can be a drug administration device used by the same patient as the first drug administration device 300a but which delivers a different drug than the first drug administration device 300a. Thus, in this way, the first drug administration device 300a can be configured to receive supplementary sensor data from the computer system 800 which indicates that the second drug administration device 300b has been used to deliver a certain drug to the patient after delivery of the drug from the first drug administration device 300a according to the first drug dosing scheme for delivery of the drug contained in the first drug administration device 300a to a patient. The first drug administration device 300a (or the computer system 800, in embodiments in which the computer system 800 determines and sends the second dosing scheme to the first drug administration device 300a) can be configured to then determine that the second drug dosing scheme should deliver the drug at a set time after the second drug administration device 300b was used to deliver the other drug to the patient in order to avoid interference between the drugs of the first and second drug administration devices 300a, 300b. Alternatively, the first drug administration device 300a (or the computer system 800, in embodiments in which the computer system 800 determines and sends the second dosing scheme to the first drug administration device 300a) can be configured to determine that the second drug dosing scheme should deliver the drug to the patient at the same time, or shortly after, the second drug administration device 300b delivers its drug to the patient because the two drugs have a beneficial effect when delivered together. If the drug administration system includes one or more additional drug administration devices, each of the additional drug administration devices can also be used by the patient and be used for the first drug administration's second dosing scheme similar to the second drug administration device 300b. This may further assist in optimizing the second drug dosing scheme for the patient who is using the first drug administration device 300a.

The second drug administration device 300b can instead be a drug administration device used by another patient (e.g., a different patient than the patient using the first drug administration device 300a) such that the first drug administration device 300a can be configured to receive supplementary sensor data related to drug administration to another patient and the other patient's response to other drug dosing scheme(s) used by the second drug administration device 300b and use this supplementary sensor data to determine the second drug dosing scheme (or the computer system 800, in embodiments in which the computer system 800 determines and sends the second dosing scheme to the first drug administration device 300a) that is optimized for the patient who is using the first drug administration device 300a. In this manner the first drug administration device 300a can be configured to implement an adjusted dosing scheme for the patient that benefits from the experience of another patient. This can speed up the drug dosing scheme optimization process. If the drug administration system includes one or more additional drug administration devices, each of the additional drug administration devices can also be used by a patient different than the patient using the first drug administration device 300a and be used for the first drug administration's second dosing scheme similar to the second drug administration device 300b. This may further assist in optimizing the current drug dosing scheme for the patient using the first drug administration device 300a.

The at least one sensor associated with the first drug administration device 300a in this illustrated embodiment includes a location sensor 98a that is configured and used similar to the location sensor 98 (Fig. 5B) discussed above that is configured to obtain sensor data relating to geographical location of the drug administration device 300a, which is an inhaler in this illustrated embodiment (e.g., the inhaler 300 of Fig. 3). The at least one sensor associated with the first drug administration device 300a in this illustrated embodiment also includes an environment sensor 94a that is configured and used similar to the location sensor 94 (Fig. 5B) discussed above that is configured to obtain data relating to an environment in which the drug administration device 300a is located, which in this illustrated embodiment includes a pollen level of the environment in which the first drug administration device 300a is present. The at least one sensor associated with the second drug administration device 300b in this illustrated embodiment similarly includes a location sensor 98b that is configured and used similar to the location sensor 98 (Fig. 5B) and includes an environment sensor 94b that is configured and used similar to the location sensor 94 (Fig. 5B) and in this illustrated embodiment includes a pollen level of the environment in which the second drug administration device 300b is present. The second drug administration device 300b is an inhaler in this illustrated embodiment (e.g., the inhaler 300 of Fig. 3). As will be appreciated by a person skilled in the art, pollen level (e.g., pollen count) may be particularly important for patients using an inhaler.

Thus, in this illustrated embodiment, the second drug dosing scheme for the first drug administration device 300a is configured to be determined dependent on dosing data from the computer system 800, on sensor data relating to the location of the first inhaler 300a, on sensor data relating to the pollen level of the environment in which the first inhaler 300a is present, on sensor data relating to the location of the second inhaler 300b, and sensor data relating to the pollen level of the environment in which the second inhaler 300b is present. Thus, for example, if the sensor data indicates that the first inhaler 300a is moving towards a location near that of the second inhaler 300b and that the pollen count is higher at the location of the second inhaler 300b than that of the first inhaler 300a, the first inhaler 300a can be configured to determine the second drug dosing scheme with a higher dose than the first drug dosing scheme to pre-emptively combat the higher pollen level the patient associated with the first drug administration device 300a may experience and thus improve patient comfort and outcomes.

In other embodiments, the at least one sensor associated with each of the first and second drug administration devices 300a, 300b can include additional or alternative sensors, such as one or more sensors including a patient monitoring sensor configured to obtain supplementary sensor data relating to a condition of a patient, a device sensor configured to obtain supplementary sensor data relating to the drug administration device, an environment sensor configured to obtain supplementary sensor data relating to the environment (other than pollen level, which is already accounted for by the first environment sensor 94a for the first drug administration device 300a and by the second environment sensor 94b for the second drug administration device 300b) in which the drug administration device is present, and a location sensor configured to obtain supplementary sensor data relating to the geographical location of the drug administration device. The patient monitoring sensor, the device sensor, the environment sensor, and the location sensor can each have any of the features discussed herein in relation to like-named sensors.

The second drug administration device 300b can include a user interface (e.g., the user interface 80 of Fig. 5B discussed above) configured to obtain supplementary user input data, and the second drug administration device 300b can be configured to communicate the supplementary user input data to the computer system 800, e.g., by the second communications interface 99b communicating with the computer system's communications interface 899, which can then either send the supplementary user input data to the first drug administration device 300a or use the supplementary user input data to determine the second drug dosing scheme and send the determined second drug dosing scheme to the first drug administration device 300a. This user interface of the second drug administration device 300b can be present in addition to or instead of the at least one sensor 94b, 98b configured to obtain supplementary sensor data described herein. The second drug administration device 300b being configured to receive and transmit user input enables the drug administration system to determine the second drug dosing scheme dependent on user input data relating to the second drug administration device 300b when determining the second drug dosing scheme. This user input data can relate to factor(s) that affect a patient's response and/or perceived response to a drug dosing scheme, as discussed herein.

In this illustrated embodiment of Fig. 15, each of the first drug administration device 300a and second drug administration device 300b includes an inhaler, e.g., the inhaler 300 of Fig. 3, but other types of drug administration devices as described herein can be used for either or both of the drug administration devices 300a, 300b (and any other drug administration devices included in the drug administration system) while the drug administration system is otherwise configured and used similar to the drug administration system of Fig. 15. For example, each of the first and second drug administration devices can include an infusion pump, e.g., the infusion pump 200 of Fig. 2. The first infusion pump can be configured to deliver insulin to a patient, and the second infusion pump can be delivered to deliver glucagon to the same patient. Each infusion pump can include a patient sensor configured to obtain sensor data relating to blood glucose level of its associated patient. Thus, the first infusion pump can be configured to determine a second drug dosing scheme to maintain a steady level of glucose in the blood based, at least in part, on supplementary sensor data obtained by the second infusion pump's patient sensor.

The drug administration system of Fig. 15 can also include at least one external sensor that is not associated with either of the first and second drug administration devices 300a, 300b and that is configured to collect external sensor data and communicate the external sensor data to the computer system 800. The second drug dosing scheme for the first drug administration device 300b can be further dependent on the external sensor data received from the computer system 800. Taking the external sensor data into account allows the first drug administration device 300a (or the computer system 800, in embodiments in which the computer system 800 determines and sends the second dosing scheme to the first drug administration device 300a) to determine the second dosing scheme dependent on additional data. As a result, more data can be used in order to determine the second drug dosing scheme, and the second drug dosing scheme can consequently be better optimized and result in improved patient outcomes. The at least one external sensor can include any of a variety of types of sensors. For example, the at least one external sensor can include an environment sensor that detects air quality in a particular location and communicates the sensor data to the computer system 800.

As discussed above, at least one sensor configured to sense data that can be used in determining a drug dosing scheme can be a part of a drug administration device. Fig. 11A illustrates one embodiment of such a device, the autoinjector 100 that includes the at least one patient monitoring sensor 91. Figs. 16A and 16B illustrate another embodiment of such a device, an autoinjector 900 (e.g., the autoinjector 100 of Fig. 1) that includes at least one patient monitoring sensor 991. The at least one patient monitoring sensor 991 in this illustrated embodiment includes a temperature sensor configured to measure a body temperature of a patient 902. The temperature sensor 991 in this illustrated embodiment is a temporal scanner configured to be placed against a forehead to measure body temperature, as shown for example in Fig. 16A in which the temperature sensor 991 is placed on a forehead of the patient 902. However, other types of temperature sensors can be used for the temperature sensor 991.

The autoinjector 900 also includes a user interface 980 in the form of a display configured to display information thereon to a user. The user interface 980 in the example of Fig. 16B shows the patient's measured temperature as 98.6 degrees Fahrenheit. As will be appreciated by a person skilled in the art, the user interface 980 can be part of the temporal scanner 991.

The effectiveness of some drugs can be affected if a patient body temperature's is above normal, e.g., above 98.6 degrees Fahrenheit. The patient's body temperature as measured by the temperature sensor 991 can be used by a computer system 800 to determine a drug dosing scheme for delivering a drug to the patient 902 using the autoinjector 900. The drug dosing scheme can be the first drug dosing scheme, the second drug dosing scheme, or a drug dosing scheme subsequent to the second drug dosing scheme. As discussed above, the computer system 800 can be included in the autoinjector 900 or can be external to the autoinjector 900 and in electronic communication therewith. A user of the autoinjector 900 can be instructed to take the patient's temperature prior to delivering drug from the autoinjector 900 to help ensure that the drug dosing scheme is adjusted, if needed, by the computer system 800 prior to drug delivery based on the sensed patient body temperature. The user can be so instructed in any of a variety of ways, such as verbally by a medical care professional, in the autoinjector's Instructions For Use (IFU), on a printed prescription label on the autoinjector's packaging, via the user interface 980 (and/or other user interface of the autoinjector 900), etc.

In an exemplary embodiment, the autoinjector 900 is configured to operate a device operation prevention mechanism of the autoinjector 900 (e.g., the device operation prevention mechanism 140 of Fig. 1) to allow the autoinjector 900 to be operated according to the drug dosing scheme if the temperature sensor 991 detects that the temperature of the patient 902 is below a predetermined high threshold that corresponds to a temperature above which effectiveness of the drug may be adversely affected if delivered to the patient 902 having that temperature or greater. One example of a predetermined high threshold is normal body temperature of 98.6 degrees Fahrenheit. The autoinjector 900 is also configured to operate a device operation prevention mechanism of the autoinjector 900 (e.g., the device operation prevention mechanism 140 of Fig. 1) to prevent the autoinjector 900 from being operated before the drug is delivered according to the drug dosing scheme if the temperature sensor 991 detects that the temperature of the patient 902 is equal to or above the predetermined high threshold. The user interface 980 (or other user interface of the autoinjector 900) can be configured to provide notification to the user that the autoinjector 900 is temporarily disabled from use due to the patient's current body temperature. The user can be instructed to take the patient's temperature again after each time the patient's temperature is measured by the temperature sensor 991 to be equal to or above the predetermined high threshold to allow the autoinjector 900 to be enabled for operation once the patient's temperature is measured to be below the predetermined high threshold. The user can be so instructed in any of a variety of ways, such as in the autoinjector's IFU, on a printed prescription label on the autoinjector's packaging, via the user interface 980 (and/or other user interface of the autoinjector 900), etc. The user can be instructed to wait a certain amount of time before re-taking the patient's temperature.

A patient's body temperature may be a biometric indicator that is important to understanding a drug's long term effects on the patient, as will be appreciated by a person skilled in the art. The patient's body temperature as measured by the temperature sensor 991 just prior to drug delivery can thus be useful in evaluating the long term effects of the drug delivered to the patient 902 using the autoinjector 900. Thus, in some embodiments, the patient's temperature as sensed by the temperature sensor 991 can be used by the computer system 800 and/or by a health care professional in determining a drug dosing scheme and in evaluating the drug's long term effects by correlating the patient's measured body temperature with each delivery of the drug to the patient from the autoinjector 900 and any other autoinjectors 900. In some embodiments, the patient's temperature as sensed by the temperature sensor 991 may not be used at all in determining a drug dosing scheme but may be used by the computer system 800 and/or by a health care professional in evaluating the drug's long term effects by correlating the patient's measured body temperature with each delivery of the drug to the patient from the autoinjector 900 and any other autoinjectors 900.

All of the devices and systems disclosed herein can be designed to be disposed of after a single use, or they can be designed to be used multiple times. In either case, however, the devices can be reconditioned for reuse after at least one use. Reconditioning can include any combination of the steps of disassembly of the devices, followed by cleaning or replacement of particular pieces, and subsequent reassembly. In particular, the devices can be disassembled, and any number of the particular pieces or parts of the device can be selectively replaced or removed in any combination. Upon cleaning and/or replacement of particular parts, the devices can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

It can be preferred that devices disclosed herein be sterilized before use. This can be done by any number of ways known to those skilled in the art including beta or gamma radiation, ethylene oxide, steam, and a liquid bath (e.g., cold soak). An exemplary embodiment of sterilizing a device including internal circuitry is described in more detail in U.S. Pat. Pub. No. 2009/0202387 published August 13, 2009 and entitled "System And Method Of Sterilizing An Implantable Medical Device." It is preferred that device, if implanted, is hermetically sealed. This can be done by any number of ways known to those skilled in the art.

The present disclosure has been described above by way of example only within the context of the overall disclosure provided herein. It will be appreciated that modifications within the scope of the claims may be made without departing from the overall scope of the present disclosure.

## Claims

1. A drug administration system, comprising:
a drug administration device (100, 200, 300, 400, 500, 900), wherein the drug administration device comprises a communications interface configured to communicate with a communications interface of a computer system (700, 800);
at least one sensor configured to obtain sensor data and communicate the sensor data to the drug administration device; and wherein:
the drug administration device (100, 200, 300, 400, 500, 900) is configured to:
utilize a first drug dosing scheme when a drug is delivered from the drug administration device to a patient;
determine a second drug dosing scheme for delivering the drug from the drug administration device to the patient, the second drug dosing scheme being dependent on:
dosing data from the computer system (700, 800); and
the sensor data from the at least one sensor, the sensor data being obtained by the at least one sensor during and/or after the delivery of the drug according to the first drug dosing scheme; and
utilize the second drug dosing scheme when the drug is delivered from the drug administration device to the patient; and wherein the at least one sensor comprises one or more of:
an environment sensor (94) configured to obtain sensor data relating to an environment in which the drug administration device is present; and
a location sensor (98) configured to obtain sensor data relating to geographical location of the drug administration device.

2. A drug administration system, comprising:
a drug administration device (100, 200, 300, 400, 500, 900), wherein the drug administration device comprises a communications interface configured to communicate with a communications interface of a computer system;
at least one user interface (80, 280) configured to obtain user input data and communicate the user input data to the drug administration device; and wherein:
the drug administration device (100, 200, 300, 400, 500, 900) is configured to:
utilize a first drug dosing scheme when a drug is delivered from the drug administration device to a patient;
determine a second drug dosing scheme for delivering the drug from the drug administration device to the patient, the second drug dosing scheme being dependent on:
dosing data from the computer system; and
the user input data from the at least one user interface, the user input data being obtained by the user interface during and/or after the delivery of the drug from the drug administration device according to the first drug dosing scheme; and
utilize the second drug dosing scheme when the drug is delivered from the drug administration device to the patient ; and wherein the system further comprises
at least one sensor configured to obtain sensor data and communicate the sensor data to the drug administration device; and
wherein: the drug administration device (100, 200, 300, 400, 500, 900) is configured to determine the second drug dosing scheme further dependent on the sensor data from the at least one sensor, the sensor data being obtained by the at least one sensor during and/or after the delivery of the drug from the drug administration device according to the first drug dosing scheme, wherein the at least one sensor comprises one or more of:
an environment sensor (94) configured to obtain sensor data relating to an environment in which the drug administration device is present; and
a location sensor (98) configured to obtain sensor data relating to geographical location of the drug administration device.

3. The drug administration system of claim 1 or claim 2, wherein the drug administration device (100, 200, 300, 400, 500, 900) is configured to determine the first drug dosing scheme for delivering the drug from the drug administration device to the patient before utilizing the first drug dosing scheme when the drug is delivered from the drug administration device to the patient.

4. The drug administration system of any one of claims 1 to 3, wherein each of the first and second drug dosing schemes each specify one or more of the following drug dosing parameters:
drug delivery rate;
drug delivery time and/or date;
drug delivery volume; and
drug delivery duration.

5. The drug administration system of any one of the preceding claims, wherein the at least one sensor further comprises a patient monitoring sensor (91) configured to obtain sensor data relating to a condition of the patient a device sensor (92) configured to obtain sensor data relating to the drug administration device; .

6. The drug administration system of any one of claims 1 to 5, further comprising:
at least one external sensor configured to obtain external sensor data and communicate the external sensor data to the computer system; and wherein
the second drug dosing scheme is further determined dependent on external sensor data from the computer system.

7. The drug administration system of any one of claims 1 to 6, further comprising:
a second drug administration device (100, 200, 300, 400, 500, 900), wherein the second drug administration device comprises a communications interface configured to communicate with the communications interface of the computer system (700, 800);
at least one sensor configured to obtain supplementary sensor data and communicate the supplementary sensor data to the second drug administration device; and wherein
the second drug dosing scheme is further determined dependent on the supplementary sensor data from the computer system (700, 800).

8. The drug administration system of any one of claims 1 to 6, further comprising:
a second drug administration device (100, 200, 300, 400, 500, 900), wherein the second drug administration device comprises a communications interface configured to communicate with the communications interface of the computer system (700, 800); and
at least one user interface (80, 280) configured to obtain supplementary user input data and communicate the supplementary user input data to the second drug administration device; and wherein
the second drug dosing scheme is further determined dependent on the supplementary user input data from the computer system (700, 800).

9. The drug administration system of claim 8, further comprising at least one sensor configured to obtain supplementary sensor data and communicate the supplementary sensor data to the second drug administration device; and
wherein the second drug dosing scheme is further determined dependent on the supplementary sensor data from the computer system.

10. The drug administration system of claim 7 or 9, wherein the at least one sensor configured to obtain supplementary sensor data comprises one or more of:
a patient monitoring sensor (91) configured to obtain supplementary sensor data relating to a condition of a patient;
a device sensor (92) configured to obtain supplementary sensor data relating to the second drug administration device;
an environment sensor (94) configured to obtain supplementary sensor data relating to an environment in which the second drug administration device is present; and
a location sensor (98) configured to obtain supplementary sensor data relating to geographical location of the second drug administration device.

11. The drug administration system of any one of claims 1 to 10, wherein the drug administration device is an autoinjector (100, 900); and wherein
the first and second drug dosing schemes each specify one or more of the following dosing parameters:
a discharge nozzle advance depth of a discharge nozzle during delivery of the drug to the patient;
a discharge nozzle velocity of the discharge nozzle during delivery of the drug to the patient; and
a discharge nozzle acceleration of the discharge nozzle during delivery of the drug to the patient.

12. The drug administration system of any one of claims 1 to 10, wherein the drug administration device is an inhaler or an infusion pump.

13. The drug administration system of claim 3, or any one of 4 to 12 when dependent on claim 3, wherein the first drug dosing scheme is determined dependent on dosing data communicated from the computer system.

14. The drug administration system of any one of claims 1 to 13, wherein:
the first and second drug dosing schemes specify one or more of the following drug dosing parameters:
drug delivery rate;
drug delivery volume; and
drug delivery duration; and wherein,
a value of one or more of the parameters in the first drug dosing scheme is different than a value of the corresponding parameter in the second drug dosing scheme.

15. The drug administration system of any preceding claim, wherein the drug comprises at least one of infliximab, golimumab, ustekinumab, daratumumab, guselkumab, epoetin alfa, risperidone, esketamine, ketamine, and paliperidone palmitate.

## Patentansprüche

1. Arzneimittelverabreichungssystem, umfassend:
eine Arzneimittelverabreichungsvorrichtung (100, 200, 300, 400, 500, 900), wobei die Arzneimittelverabreichungsvorrichtung eine Kommunikationsschnittstelle umfasst, die konfiguriert ist, um mit einer Kommunikationsschnittstelle eines Computersystems (700, 800) zu kommunizieren;
mindestens einen Sensor, der konfiguriert ist, um Sensordaten zu erhalten und die Sensordaten an die Arzneimittelverabreichungsvorrichtung zu kommunizieren; und wobei:
die Arzneimittelverabreichungsvorrichtung (100, 200, 300, 400, 500, 900) konfiguriert ist zum:
Nutzen eines ersten Arzneimitteldosierungsschemas, wenn ein Arzneimittel von der Arzneimittelverabreichungsvorrichtung an einen Patienten abgegeben wird;
Bestimmen eines zweiten Arzneimitteldosierungsschemas zum Abgeben des Arzneimittels von der Arzneimittelverabreichungsvorrichtung an den Patienten, wobei das zweite Arzneimitteldosierungsschema in Abhängigkeit steht von:
Dosierungsdaten von dem Computersystem (700, 800); und
den Sensordaten von dem mindestens einen Sensor, wobei die Sensordaten durch den mindestens einen Sensor während und/oder nach der Verabreichung des Arzneimittels gemäß dem ersten Arzneimitteldosierungsschema erhalten werden; und
Nutzen des zweiten Arzneimitteldosierungsschemas, wenn das Arzneimittel aus der Arzneimittelverabreichungsvorrichtung an den Patienten abgegeben wird; und wobei der mindestens eine Sensor eines oder mehrere umfasst von:
einem Umgebungssensor (94), der konfiguriert ist, um Sensordaten bezüglich einer Umgebung zu erhalten, in der sich die Arzneimittelverabreichungsvorrichtung befindet; und
einem Standortsensor (98), der konfiguriert ist, um Sensordaten bezüglich des geografischen Standorts der Arzneimittelverabreichungsvorrichtung zu erhalten.

2. Arzneimittelverabreichungssystem, umfassend:
eine Arzneimittelverabreichungsvorrichtung (100, 200, 300, 400, 500, 900), wobei die Arzneimittelverabreichungsvorrichtung eine Kommunikationsschnittstelle umfasst, die konfiguriert ist, um mit einer Kommunikationsschnittstelle eines Computersystems zu kommunizieren;
mindestens eine Benutzerschnittstelle (80, 280), die konfiguriert ist, um Benutzereingabedaten zu erhalten und die Benutzereingabedaten an die Arzneimittelverabreichungsvorrichtung zu kommunizieren; und wobei:
die Arzneimittelverabreichungsvorrichtung (100, 200, 300, 400, 500, 900) konfiguriert ist zum:
Nutzen eines ersten Arzneimitteldosierungsschemas, wenn ein Arzneimittel von der Arzneimittelverabreichungsvorrichtung an einen Patienten abgegeben wird;
Bestimmen eines zweiten Arzneimitteldosierungsschemas zum Abgeben des Arzneimittels von der Arzneimittelverabreichungsvorrichtung an den Patienten, wobei das zweite Arzneimitteldosierungsschema in Abhängigkeit steht von:
Dosierungsdaten aus dem Computersystem; und
den Benutzereingabedaten von der mindestens einen Benutzerschnittstelle, wobei die Benutzereingabedaten durch die Benutzerschnittstelle während und/oder nach der Abgabe des Arzneimittels aus der Arzneimittelverabreichungsvorrichtung gemäß dem ersten Arzneimitteldosierungsschema erhalten werden; und
Nutzen des zweiten Arzneimitteldosierungsschemas, wenn das Arzneimittel aus der Arzneimittelverabreichungsvorrichtung an den Patienten abgegeben wird; und wobei das System ferner umfasst
mindestens einen Sensor, der konfiguriert ist, um Sensordaten zu erhalten und die Sensordaten an die Arzneimittelverabreichungsvorrichtung zu kommunizieren; und
wobei: die Arzneimittelverabreichungsvorrichtung (100, 200, 300, 400, 500, 900) konfiguriert ist, um das zweite Arzneimitteldosierungsschema ferner in Abhängigkeit von den Sensordaten von dem mindestens einen Sensor zu bestimmen, wobei die Sensordaten durch den mindestens einen Sensor während und/oder nach der Abgabe des Arzneimittels aus der Arzneimittelverabreichungsvorrichtung gemäß dem ersten Arzneimitteldosierungsschema erhalten werden, wobei der mindestens eine Sensor eines oder mehrere umfasst von:
einem Umgebungssensor (94), der konfiguriert ist, um Sensordaten bezüglich einer Umgebung zu erhalten, in der sich die Arzneimittelverabreichungsvorrichtung befindet; und
einem Standortsensor (98), der konfiguriert ist, um Sensordaten bezüglich des geografischen Standorts der Arzneimittelverabreichungsvorrichtung zu erhalten.

3. Arzneimittelverabreichungssystem nach Anspruch 1 oder 2, wobei die Arzneimittelverabreichungsvorrichtung (100, 200, 300, 400, 500, 900) konfiguriert ist, um das erste Arzneimitteldosierungsschema zum Abgeben des Arzneimittels von der Arzneimittelverabreichungsvorrichtung an den Patienten zu bestimmen, bevor sie das erste Arzneimitteldosierungsschema nutzt, wenn das Arzneimittel von der Arzneimittelverabreichungsvorrichtung an den Patienten abgegeben wird.

4. Arzneimittelverabreichungssystem nach einem der Ansprüche 1 bis 3, wobei jedes des ersten und des zweiten Arzneimitteldosierungsschemas jeweils einen oder mehrere der folgenden Arzneimitteldosierungsparameter spezifizieren:
Arzneimittelabgaberate;
Uhrzeit und/oder Datum einer Arzneimittelabgabe;
Arzneimittelabgabevolumen; und Dauer der Arzneimittelabgabe.

5. Arzneimittelverabreichungssystem nach einem der vorstehenden Ansprüche, wobei der mindestens eine Sensor ferner einen Patientenüberwachungssensor (91) umfasst, der konfiguriert ist, um Sensordaten bezüglich eines Zustands des Patienten zu erhalten, einen Vorrichtungssensor (92), der konfiguriert ist, um Sensordaten bezüglich der Arzneimittelverabreichungsvorrichtung zu erhalten .

6. Arzneimittelverabreichungssystem nach einem der Ansprüche 1 bis 5, ferner umfassend:
mindestens einen externen Sensor, der konfiguriert ist, um externe Sensordaten zu erhalten und die externen Sensordaten an das Computersystem zu kommunizieren; und wobei
das zweite Arzneimitteldosierungsschema ferner in Abhängigkeit von externen Sensordaten aus dem Computersystem bestimmt wird.

7. Arzneimittelverabreichungssystem nach einem der Ansprüche 1 bis 6, ferner umfassend:
eine zweite Arzneimittelverabreichungsvorrichtung (100, 200, 300, 400, 500, 900), wobei die zweite Arzneimittelverabreichungsvorrichtung eine Kommunikationsschnittstelle umfasst, die konfiguriert ist, um mit der Kommunikationsschnittstelle des Computersystems (700, 800) zu kommunizieren;
mindestens einen Sensor, der konfiguriert ist, um ergänzende Sensordaten zu erhalten und die ergänzenden Sensordaten an die zweite Arzneimittelverabreichungsvorrichtung zu kommunizieren; und wobei
das zweite Arzneimitteldosierungsschema ferner in Abhängigkeit von den ergänzenden Sensordaten von dem Computersystem (700, 800) bestimmt wird.

8. Arzneimittelverabreichungssystem nach einem der Ansprüche 1 bis 6, ferner umfassend:
eine zweite Arzneimittelverabreichungsvorrichtung (100, 200, 300, 400, 500, 900), wobei die zweite Arzneimittelverabreichungsvorrichtung eine Kommunikationsschnittstelle umfasst, die konfiguriert ist, um mit der Kommunikationsschnittstelle desComputersystems (700, 800) zu kommunizieren; und
mindestens eine Benutzerschnittstelle (80, 280), die konfiguriert ist, um ergänzende Benutzereingabedaten zu erhalten und die ergänzenden Benutzereingabedaten an die zweite Arzneimittelverabreichungsvorrichtung zu kommunizieren; und wobei
das zweite Arzneimitteldosierungsschema ferner in Abhängigkeit von den ergänzenden Benutzereingabedaten aus dem Computersystem (700, 800) bestimmt wird.

9. Arzneimittelverabreichungssystem nach Anspruch 8, ferner umfassend mindestens einen Sensor, der konfiguriert ist, um ergänzende Sensordaten zu erhalten und die ergänzenden Sensordaten an die zweite Arzneimittelverabreichungsvorrichtung zu kommunizieren; und
wobei das zweite Arzneimitteldosierungsschema ferner in Abhängigkeit von den ergänzenden Sensordaten des Computersystems bestimmt wird.

10. Arzneimittelverabreichungssystem nach Anspruch 7 oder 9, wobei der mindestens eine Sensor, der konfiguriert ist, um ergänzende Sensordaten zu erhalten, eines oder mehrere umfasst von:
einem Patientenüberwachungssensor (91), der konfiguriert ist, um ergänzende Sensordaten bezüglich eines Zustands eines Patienten zu erhalten;
einem Vorrichtungssensor (92), der konfiguriert ist, ergänzende Sensordaten bezüglich der zweiten Arzneimittelverabreichungsvorrichtung zu erhalten;
einem Umgebungssensor (94), der konfiguriert ist, um ergänzende Sensordaten bezüglich einer Umgebung zu erhalten, in der die zweite Arzneimittelverabreichungsvorrichtung vorhanden ist; und
einem Standortsensor (98), der konfiguriert ist, um ergänzende Sensordaten bezüglich des geografischen Standorts der zweiten Arzneimittelverabreichungsvorrichtung zu erhalten.

11. Arzneimittelverabreichungssystem nach einem der Ansprüche 1 bis 10, wobei die Arzneimittelverabreichungsvorrichtung ein Autoinjektor (100, 900) ist; und wobei
das erste und das zweite Arzneimitteldosierungsschema jeweils einen oder mehrere der folgenden Dosierungsparameter spezifizieren:
eine Ausstoßdüsenvorschubtiefe einer Ausstoßdüse während der Abgabe des Arzneimittels an den Patienten;
eine Ausstoßdüsengeschwindigkeit der Ausstoßdüse während der Abgabe des Arzneimittels an den Patienten; und
eine Ausstoßdüsenbeschleunigung der Ausstoßdüse während der Abgabe des Arzneimittels an den Patienten.

12. Arzneimittelverabreichungssystem nach einem der Ansprüche 1 bis 10, wobei die Arzneimittelverabreichungsvorrichtung ein Inhalator oder eine Infusionspumpe ist.

13. Arzneimittelverabreichungssystem nach Anspruch 3 oder einem der Ansprüche 4 bis 12 in Abhängigkeit von Anspruch 3, wobei das erste Arzneimitteldosierungsschema in Abhängigkeit von den Dosierungsdaten bestimmt wird, die von dem Computersystem kommuniziert werden.

14. Arzneimittelverabreichungssystem nach einem der Ansprüche 1 bis 13, wobei:
das erste und das zweite Arzneimitteldosierungsschema einen oder mehrere der folgenden Arzneimitteldosierungsparameter spezifizieren:
Arzneimittelabgaberate;
Arzneimittelabgabevolumen; und
Dauer der Arzneimittelverabreichung; und wobei,
ein Wert eines oder mehrerer Parameter in dem ersten Arzneimitteldosierungsschema sich von einem Wert des entsprechenden Parameters in dem zweiten Arzneimitteldosierungsschema unterscheidet.

15. Arzneimittelverabreichungssystem nach einem der vorstehenden Ansprüche, wobei das Arzneimittel mindestens eines von Infliximab, Golimumab, Ustekinumab, Daratumumab, Guselkumab, Epoetin alfa, Risperidon, Esketamin, Ketamin und Paliperidonpalmitat umfasst.

## Revendications

1. Système d'administration de médicament, comprenant :
un dispositif d'administration de médicament (100, 200, 300, 400, 500, 900), dans lequel le dispositif d'administration de médicament comprend une interface de communications configurée pour communiquer avec une interface de communications d'un système informatique (700, 800) ;
au moins un capteur configuré pour obtenir des données de capteur et communiquer les données de capteur au dispositif d'administration de médicament ; et dans lequel :
le dispositif d'administration de médicament (100, 200, 300, 400, 500, 900) est configuré pour :
utiliser un premier schéma de dosage de médicament lorsqu'un médicament est délivré du dispositif d'administration de médicament à un patient ;
déterminer un second schéma de dosage de médicament permettant de délivrer le médicament du dispositif d'administration de médicament au patient, le second schéma de dosage de médicament dépendant de :
données de dosage provenant du système informatique (700, 800) ; et
des données de capteur provenant de l'au moins un capteur, les données de capteur étant obtenues par l'au moins un capteur pendant et/ou après la délivrance du médicament selon le premier schéma de dosage de médicament ; et
utiliser le second schéma de dosage de médicament lorsque le médicament est délivré du dispositif d'administration de médicament au patient ; et dans lequel l'au moins un capteur comprend un ou plusieurs parmi :
un capteur d'environnement (94) configuré pour obtenir des données de capteur se rapportant à un environnement dans lequel le dispositif d'administration de médicament est présent ; et
un capteur de localisation (98) configuré pour obtenir des données de capteur se rapportant à une localisation géographique du dispositif d'administration de médicament.

2. Système d'administration de médicament, comprenant :
un dispositif d'administration de médicament (100, 200, 300, 400, 500, 900), dans lequel le dispositif d'administration de médicament comprend une interface de communications configurée pour communiquer avec une interface de communications d'un système informatique ;
au moins une interface utilisateur (80, 280) configurée pour obtenir des données d'entrée utilisateur et communiquer les données d'entrée utilisateur au dispositif d'administration de médicament ; et dans lequel :
le dispositif d'administration de médicament (100, 200, 300, 400, 500, 900) est configuré pour :
utiliser un premier schéma de dosage de médicament lorsqu'un médicament est délivré du dispositif d'administration de médicament à un patient ;
déterminer un second schéma de dosage de médicament permettant de délivrer le médicament du dispositif d'administration de médicament au patient, le second schéma de dosage de médicament dépendant de :
données de dosage provenant du système informatique ; et
des données d'entrée utilisateur provenant de l'au moins une interface utilisateur, les données d'entrée utilisateur étant obtenues par l'interface utilisateur pendant et/ou après la délivrance du médicament à partir du dispositif d'administration de médicament selon le premier schéma de dosage de médicament ; et
utiliser le second schéma de dosage de médicament lorsque le médicament est délivré du dispositif d'administration de médicament au patient ; et dans lequel le système comprend en outre
au moins un capteur configuré pour obtenir des données de capteur et communiquer les données de capteur au dispositif d'administration de médicament ; et
dans lequel : le dispositif d'administration de médicament (100, 200, 300, 400, 500, 900) est configuré pour déterminer le second schéma de dosage de médicament en dépendance en outre des données de capteur provenant de l'au moins un capteur, les données de capteur étant obtenues par l'au moins un capteur pendant et/ou après la délivrance du médicament à partir du dispositif d'administration de médicament selon le premier schéma de dosage de médicament, dans lequel l'au moins un capteur comprend un ou plusieurs parmi :
un capteur d'environnement (94) configuré pour obtenir des données de capteur se rapportant à un environnement dans lequel le dispositif d'administration de médicament est présent ; et
un capteur de localisation (98) configuré pour obtenir des données de capteur se rapportant à une localisation géographique du dispositif d'administration de médicament.

3. Système d'administration de médicament selon la revendication 1 ou la revendication 2, dans lequel le dispositif d'administration de médicament (100, 200, 300, 400, 500, 900) est configuré pour déterminer le premier schéma de dosage de médicament permettant de délivrer le médicament du dispositif d'administration de médicament au patient avant d'utiliser le premier schéma de dosage de médicament lorsque le médicament est délivré du dispositif d'administration de médicament au patient.

4. Système d'administration de médicament selon l'une quelconque des revendications 1 à 3, dans lequel chacun des premier et second schémas de dosage de médicament spécifie un ou plusieurs des paramètres de dosage de médicament suivants :
débit de délivrance de médicament ;
heure et/ou date de délivrance de médicament ;
volume de délivrance de médicament ; et
durée de délivrance de médicament.

5. Système d'administration de médicament selon l'une quelconque des revendications précédentes, dans lequel l'au moins un capteur comprend en outre un capteur de surveillance de patient (91) configuré pour obtenir des données de capteur se rapportant à une condition du patient, un capteur de dispositif (92) configuré pour obtenir des données de capteur se rapportant au dispositif d'administration de médicament ; .

6. Système d'administration de médicament selon l'une quelconque des revendications 1 à 5, comprenant en outre :
au moins un capteur externe configuré pour obtenir des données de capteur externe et communiquer les données de capteur externe au système informatique ; et dans lequel
le second schéma de dosage de médicament est déterminé en outre en dépendance de données de capteur externe provenant du système informatique.

7. Système d'administration de médicament selon l'une quelconque des revendications 1 à 6, comprenant en outre :
un second dispositif d'administration de médicament (100, 200, 300, 400, 500, 900), dans lequel le second dispositif d'administration de médicament comprend une interface de communications configurée pour communiquer avec l'interface de communications du système informatique (700, 800) ;
au moins un capteur configuré pour obtenir des données de capteur complémentaires et communiquer les données de capteur complémentaires au second dispositif d'administration de médicament ; et dans lequel
le second schéma de dosage de médicament est déterminé en outre en dépendance des données de capteur complémentaires provenant du système informatique (700, 800).

8. Système d'administration de médicament selon l'une quelconque des revendications 1 à 6, comprenant en outre :
un second dispositif d'administration de médicament (100, 200, 300, 400, 500, 900), dans lequel le second dispositif d'administration de médicament comprend une interface de communications configurée pour communiquer avec l'interface de communications du système informatique (700, 800) ; et
au moins une interface utilisateur (80, 280) configurée pour obtenir des données d'entrée utilisateur complémentaires et communiquer les données d'entrée utilisateur complémentaires au second dispositif d'administration de médicament ; et dans lequel
le second schéma de dosage de médicament est déterminé en outre en dépendance des données d'entrée utilisateur complémentaires provenant du système informatique (700, 800).

9. Système d'administration de médicament selon la revendication 8, comprenant en outre au moins un capteur configuré pour obtenir des données de capteur complémentaires et communiquer les données de capteur complémentaires au second dispositif d'administration de médicament ; et
dans lequel le second schéma de dosage de médicament est déterminé en outre en dépendance des données de capteur complémentaires provenant du système informatique.

10. Système d'administration de médicament selon la revendication 7 ou 9, dans lequel l'au moins un capteur configuré pour obtenir des données de capteur complémentaires comprend un ou plusieurs parmi :
un capteur de surveillance de patient (91) configuré pour obtenir des données de capteur complémentaires se rapportant à une condition d'un patient ;
un capteur de dispositif (92) configuré pour obtenir des données de capteur complémentaires se rapportant au second dispositif d'administration de médicament ;
un capteur d'environnement (94) configuré pour obtenir des données de capteur complémentaires se rapportant à un environnement dans lequel le second dispositif d'administration de médicament est présent ; et
un capteur de localisation (98) configuré pour obtenir des données de capteur complémentaires se rapportant à une localisation géographique du second dispositif d'administration de médicament.

11. Système d'administration de médicament selon l'une quelconque des revendications 1 à 10, dans lequel le dispositif d'administration de médicament est un auto-injecteur (100, 900) ; et dans lequel
les premier et second schémas de dosage de médicament spécifient chacun un ou plusieurs des paramètres de dosage suivants :
une profondeur d'avance de buse d'éjection d'une buse d'éjection pendant la délivrance du médicament au patient ;
une vitesse de buse d'éjection de la buse d'éjection pendant la délivrance du médicament au patient ; et
une accélération de buse d'éjection de la buse d'éjection pendant la délivrance du médicament au patient.

12. Système d'administration de médicament selon l'une quelconque des revendications 1 à 10, dans lequel le dispositif d'administration de médicament est un inhalateur ou une pompe à perfusion.

13. Système d'administration de médicament selon la revendication 3, ou l'une quelconque de 4 à 12 prise en dépendance de la revendication 3, dans lequel le premier schéma de dosage de médicament est déterminé en dépendance de données de dosage communiquées par le système informatique.

14. Système d'administration de médicament selon l'une quelconque des revendications 1 à 13, dans lequel :
les premier et second schémas de dosage de médicament spécifient un ou plusieurs des paramètres de dosage de médicament suivants :
débit de délivrance de médicament ;
volume de délivrance de médicament ; et
durée de délivrance de médicament ; et dans lequel,
une valeur d'un ou plusieurs des paramètres dans le premier schéma de dosage de médicament est différente d'une valeur du paramètre correspondant dans le second schéma de dosage de médicament.

15. Système d'administration de médicament selon l'une quelconque revendication précédente, dans lequel le médicament comprend au moins l'un parmi infliximab, golimumab, ustékinumab, daratumumab, guselkumab, époétine alfa, rispéridone, eskétamine, kétamine et palmitate de palipéridone.
